(19) Europäisches Patentamt
European Patent Office
Office européen des brevets

(11) Publication number: 0 110 442
B1

(12) **EUROPEAN PATENT SPECIFICATION**

(45) Date of publication of patent specification: **06.04.88**

(51) Int. Cl.4: **C 07 C 127/19,** C 07 D 317/28, C 07 D 339/06, A 01 N 47/30 // C07C97/10

(21) Application number: **83201512.7**

(22) Date of filing: **20.10.83**

(54) **Urea compounds, intermediates and process for their preparation, herbicidal and/or fungicidal compositions, and method of combating undesired plant growth.**

(30) Priority: **03.11.82 GB 8231444**

(43) Date of publication of application: **13.06.84 Bulletin 84/24**

(45) Publication of the grant of the patent: **06.04.88 Bulletin 88/14**

(84) Designated Contracting States: **AT BE CH DE FR GB IT LI LU NL SE**

(56) References cited:
**EP-A-0 081 206**

**CHEMICAL ABSTRACTS, vol. 74, no. 11, 15th March 1971, page 310, no. 53196c, Columbus, Ohio, US; M.DZURILLA et al.: "Isothiocyanates and their synthetic producers. IX. Synthesis and study of the infrared, ultraviolet, and NMR spectra of 4'-substituted 3-isothiocyanato- and 4-isothiocyanatochalcones".**

**JOURNAL OF THE CHEMICAL SOCIETY, Perkin Transactions II, vol. 14, 1976, pages 1683-1688, London, GB; R.J.BUSHBY et al.: "Organic Anions. Part 1. Equilibration of 1,3-diarylpropenes".**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Leonard, John**
**38 Eastwood Road**
**Sittingbourne Kent (GB)**
Inventor: **Flood, Andrew**
**69 Park Road**
**Sittingbourne Kent (GB)**

(74) Representative: **Hunter, Keith Roger Ian et al**
**4 York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel herbicidal and/or fungicidal ureas.

US Specification No. 2,655,447 discloses an extremely wide class of urea derivatives having herbicidal properties. Over the twenty-five years since these compounds were disclosed, much research has been carried out into urea-type herbicides. The Applicants have now found a novel class of urea derivatives having interesting herbicidal properties, and in some cases also fungicidal properties.

EP—A—81206, which was not published at the priority date of the present invention but claims an earlier priority date, discloses that compounds of the formula A below are herbicidally active

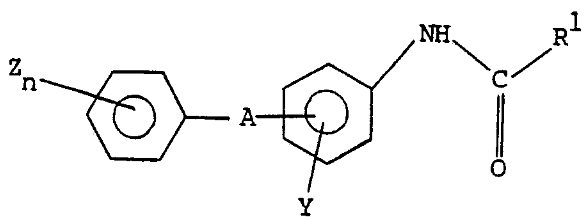

(A)

wherein $R^1$ represents $C_{1-4}$ alkoxy, alkylthio or alkyl, which is optionally substituted by halogen or $C_{1-4}$ alkoxy; or

$C_{3-6}$ cycloalkyl; or the group

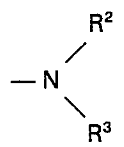

where $R^2$ and $R^3$ independently represents hydrogen, $C_{1-4}$ alkyl or alkoxy or $C_{3-6}$ cycloalkyl, or $R^2$ and $R^3$ together represent a $C_{2-6}$ alkylene group optionally substituted by methyl and optionally comprising oxygen as a chain atom;

Y represents hydrogen, halogen, methyl, methoxy or trifluoromethyl;

A represents a $C_{2-9}$ alkylene group containing a carbonyl group or a carbinol group of formula CH—OH, and optionally substituted by methyl;

Z represents hydrogen, halogen, $C_{1-6}$ alkyl, alkoxy, haloalkyl or haloalkoxy, phenyl or phenoxy;

n represents 1, 2 or 3;

and the group

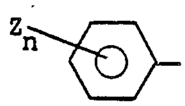

can alternatively represent a naphthyl group optionally substituted by halogen, $C_{1-4}$ alkyl or alkoxy.

Preferred compounds of formula A are stated to be those in which $R^1$ represents the group

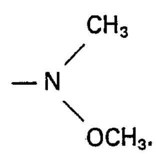

In the specific examples, the only compounds in which $R^1$ represents a dimethylamino group which were present in the first application from which EP—A—81206 claims priority are compounds in which $Z_n$ and Y both represent hydrogen and A represents the group $—CO—CH(CH_3)—CH_2—$ and $—(CH_2)_2CO—$ respectively.

Accordingly, the invention may be defined, in respect of designated states common to EP—A—81206 and the present application (namely AU, BE, CH, DE, FR, GB, IT, LI, NL), as a compound (or a composition comprising such a compound, in the case of AU) of the general formula

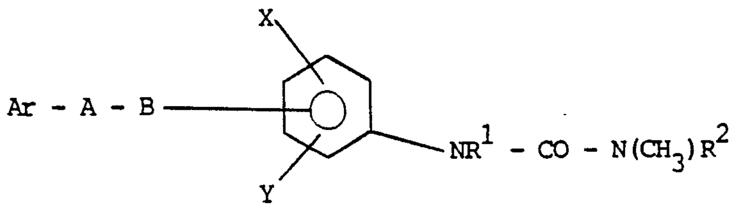

(I)

in which $R^1$ represents a hydrogen atom or an alkyl group having 1 to 4 carbon atoms; $R^2$ represents a methyl group; Ar represents an optionally substituted phenyl group or an optionally substituted heteraromatic ring containing 1 or 2 oxygen, sulphur and/or nitrogen atoms, optional substituents being selected from halogen atoms and alkyl, alkoxy, haloalkyl, haloalkoxy, alkylthio, phenyl, phenoxy, cyano, amino, alkylamino, amido, hydroxy, carboxy and alkoxycarbonyl groups, alkyl moieties in such substituents having up to 4 carbon atoms; each of X and Y independently represents a hydrogen or halogen atom or an alkyl, haloalkyl or alkoxy group; and one of A and B represents a group —CO; —$CR^3(OR^4)$— or —$CR^3(SR^4)$—, in which $R^3$ represents a hydrogen atom or an alkyl group and $R^4$ represents a hydrogen atom, an alkyl, alkenyl, alkynyl, or cycloalkyl group, or an optionally substituted phenyl or benzyl group, optional substituents being selected from halogen atoms and alkyl, alkoxy, haloalkyl, haloalkoxy, alkylthio, phenyl, phenoxy, cyano, amino, alkylamino, amido, hydroxy, carboxy and alkoxycarbonyl groups, alkyl moieties in such substituents having up to 4 carbon atoms; or an acyl group derived from a carboxylic, carbamic, sulphonic or phosphoric acid; —$C(OR^5)$ $(OR^6)$—, —$C(OR^5)(SR^6)$— or —$C(SR^5)(SR^6)$—, in which each of $R^5$ and $R^6$ independently represents an alkyl group or together represents an alkylene group; —$CH(CH_2COOR^7)$—, in which $R^7$ represents an alkyl group; or —$(CNR^8)$—, in which $R^8$ represents a group $OR^4$ or $NHR^4$; and the other of A and B represents a group —$CR^9R^{11}$—$CR^{10}R^{12}$—, in which each of $R^9$, $R^{10}$, $R^{11}$ and $R^{12}$ independently represents a hydrogen atom or an alkyl group, or $R^{11}$ and $R^{12}$ together represent a carbon-carbon bond, a —$CH_2$— group or an oxygen atom; but not including compounds of formula I wherein $R^1$, X and Y are all hydrogen atoms, Ar is unsubstituted phenyl and the linkage —A—B— is —CO—$CH(CH_3)$—$CH_2$— or —$(CH_2)_2$—CO—.

The invention may be defined, in respect of the states designated in the present application but not in EP—A—81206, (namely LU, SE), as a compound of the general formula

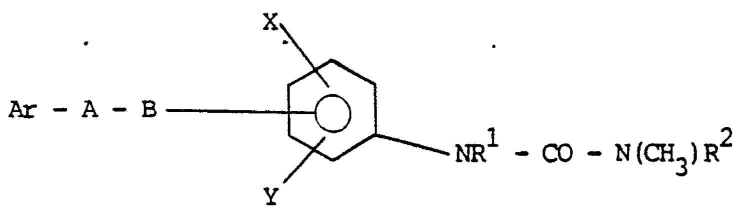

(I)

in which $R^1$ represents a hydrogen atom or an alkyl group having 1 to 4 carbon atoms; $R^2$ represents a hydrogen atom, a methoxy or a methyl group; Ar represents; an optionally substituted phenyl group or an optionally substituted heteroaromatic ring containing 1 or 2 oxygen, sulphur and/or nitrogen atoms, optional substituents being selected from halogen atoms and alkyl, alkoxy, haloalkyl, haloalkoxy, alkylthio, phenyl, phenoxy, cyano, amino, alkylamino, amido, hydroxy, carboxy and alkoxycarbonyl groups, alkyl moieties in such substituents having up to 4 carbon atoms; each of X and Y independently represents a hydrogen or halogen atom or an alkyl, haloalkyl or alkoxy group; and one of A and B represents a group —CO—; —$CR^3(OR^4)$— or —$CR^3(SR^4)$—, in which $R^3$ represents a hydrogen atom or an alkyl group and $R^4$ represents a hydrogen atom, an alkyl, alkenyl, alkynyl or cycloalkyl group, an optionally substituted phenyl or benzyl group, optional substituents being selected from halogen atoms and alkyl, alkoxy, haloalkyl, haloalkoxy, alkylthio, phenyl, phenoxy, cyano, amino, alkylamino, amido, hydroxy, carboxy and alkoxycarbonyl group, alkyl moieties in such substituents having up to 4 carbon atoms; or an acyl group derived from a carboxylic, carbamic, sulphonic or phosphoric acid; $C(OR^5)(OR^6)$—, —$C(OR^5)(SR^6)$— or —$C(SR^5)(SR^6)$—, in which each of $R^5$ and $R^6$ independently represents an alkyl group or together represents an alkylene group; —$CH(CH_2COOR^7)$— in which $R^7$ represents an alkyl group; or —$(CNR^8)$— in which $R^8$ represents a group $OR^4$ or $HNR^4$; and the other of A and B represents a group —$CR^9R^{11}$—$CR^{10}R^{12}$—, in which each of $R^9$, $R^{10}$, $R^{11}$ and $R^{12}$ independently represents a hydrogen atom or an alkyl group, or $R^{11}$ and $R^{12}$ together represents a carbon-carbon bond, a —$CH_2$— group or an oxygen atom.

In respect of the definition of the previous paragraph, $R^2$ preferably represents a methoxy or, especially, a methyl group.

The remainder of the description applies equally to each of the main definitions of the invention given above, unless otherwise stated.

Throughout this application, unless otherwise stated, any alkyl, alkenyl or alkynyl moiety preferably has up to 6, especially up to 4, carbon atoms, any cycloalkyl moiety preferably has up to 6 carbon atoms, and any alkylene moiety preferably has up to 4 carbon atoms.

Preferably $R^1$ represents a methyl group or, especially, a hydrogen atom.

Especially preferred substituents for a phenyl group are halogen atoms and alkyl, especially methyl, alkoxy, especially methoxy, and a haloalkyl, especially trifluoromethyl, groups. A phenyl group Ar is preferably unsubstituted or substituted by one or two substituents. Preferred halogen atoms comprise flourine and, especially, chlorine.

Especially preferred substituents for a heteroaromatic ring Ar are halogen atoms and alkyl, especially methyl, groups. More preferably however a heteroaromatic ring Ar is unsubstituted. The ring preferably has 5 or 6 atoms in the ring. Preferred rings are pyridine, furan, pyrrole and thiophene rings, especially pyridine rings.

An alkyl, haloalkyl or alkoxy group X or Y preferably has up to 4 carbon atoms. Preferably each of X and Y independently represents a hydrogen or halogen atom or a methyl group. More preferably, X represents a hydrogen atom and Y represents a halogen, for example chlorine, or, especially, a hydrogen atom. Most preferably both X and Y represent hydrogen atoms.

Preferably $R^3$ represents an alkyl group having 1 to 4 carbon atoms, for example a methyl group or, especially, a hydrogen atom.

An acyl group $R^4$ may, for example, have the general formula $—CO—R^{13}$, $—CO—NH_2$, $—CO—NHR^{13}$, $—CO—N(R^{13})_2$, $—SO_2—R^{13}$ or $—PO(OR^{13})_2$, in which $R^{13}$ represents an alkyl, phenyl or alkylphenyl group. Preferably $R^{13}$ represents a methyl group. An optionally substituted phenyl or benzyl group $R^4$ may for example be substituted by the preferred substituents described above for a phenyl group Ar. Preferably $R^4$ represents a hydrogen atom, an alkyl group preferably having up to 4 carbon atoms, for example a methyl, ethyl or isopropyl group, a benzyl group, an alkylcarbonyl, especially acetyl group, or a methylsulphonyl group.

Preferably each of $R^5$ and $R^6$ independently represents a methyl or ethyl group, or $R^5$ and $R^6$ together represent a dimethylene or trimethylene group. Preferably $R^7$ represents a methyl or ethyl group.

Preferably $R^8$ represents a hydroxy, a methoxy, a methoxycarbonylmethoxy or a tosylamido group.

Preferably each of $R^9$ and $R^{10}$ independently represents a methyl group or, especially, a hydrogen atom. Preferably each of $R^{11}$ and $R^{12}$ independently represents a methyl group or, especially, a hydrogen atom, or $R^{11}$ and $R^{12}$ together represent a $—CH_2—$ group or a carbon-carbon bond.

Preferably one of A and B, especially A, represents a group $—CO—$, $—CR^3(OR^4)—$, $—C(OR^5)(OR^6)—$ or $—C(SR^5)(SR^6)—$, where $R^3$, $R^4$, $R^5$ and $R^6$ have the meanings and preferred meanings given above. Especially preferred are the groups $—CO—$, $—CH(OH)—$, $—CH(OCH_3)—$, and

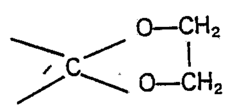

Preferably the other of A and B, especially B, represents a group $—CH{=}CH—$ or $—CH_2—CH_2—$.

The group B is preferably situated on the phenyl ring of the general formula I in the position *para* or, especially *meta* to the $—NR^1—$ group.

Depending in the various groups present in the molecule, the compounds of the general formula I may exist in the form of geometric and/or optical isomers. The invention should be understood to include all individual isomers and mixtures thereof, in particular the mixtures of the regioisomeric pairs in which A and B are interchanged.

The invention also provides a process for the preparation of a compound of the general formula I, which comprises reacting a compound of the general formula:

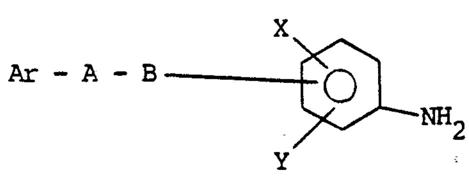

(II)

in which Ar, A, B, X and Y have the meanings given for the general formula I, with either

(a) a compound of the general formula:

$$Hal—CO—N(CH_3)R^2 \quad (III)$$

in which $R^2$ has the meaning given for the general formula I and Hal represents a halogen, especially chlorine, atom; or

(b) phosgene ($COCl_2$), to produce the corresponding isocyanate, and reacting at least part of the isocyanate with an amine of the general formula:

$$HN(CH_3)R^2 \quad (IV)$$

in which $R^2$ has the meaning given for the general formula I;

to produce a compound of the general formula I in which $R^1$ represents a hydrogen atom; and if desired, converting the resulting compound of the general formula I into any other desired compound of the general formula I.

Process (a) may be carried out with or without a solvent. Suitable solvents include hydrocarbons, and halogenated hydrocarbons, for example benzene, toluene or carbon tetrachloride. Preferably it is carried out under substantially anhydrous conditions. Since the reaction is a condensation reaction involving the elimination of hydrogen halide it is preferably carried out in the presence of a dehydrohalogenating agent. As such organic or inorganic bases are suitable; sodium acetate has been found to be especially useful when working without solvent, and organic bases such as triethylamine or pyridine are suitable when using a solvent. The reaction is preferably carried out at a temperature in the range of from 0 to 80°C, conveniently at room temperature, and the reaction mixture may be worked up by conventional means.

Process (b) is suitably carried out in the presence of a solvent, for example a hydrocarbon, e.g. toluene, at a temperature in the range of from 80 to 120°C; conveniently, the reaction may be carried out at the reflux temperature of the solvent used. If desired, the intermediate isocyanate may be isolated from the reaction mixture, but preferably it is reacted *in situ* with the amine (IV). This reaction is suitably carried out at a temperature in the range of from 0 to 30°C, conveniently at room temperature. The desired product may be isolated by any suitable method.

The starting materials of the compound of general formula II are novel compounds, and the invention therefore also provides these compounds *per se.* A compound of the general formula II may for example be prepared by reduction of a compound of the general formula:

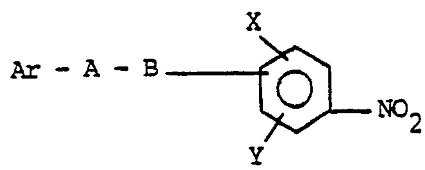

(V)

where Ar, A, B, X and Y have the meanings given for the general formula I. Any suitable reducing agent may be used, for example gaseous hydrogen over a catalyst, for example a palladium, platinum or nickel catalyst, or a hydride reducing agent, for example a complex metal hydride such as lithium aluminium hydride.

A compound of the general formula V in which one of A and B represents a —CO— group and the other represents a group $-CR^9=CR^{10}-$ may be prepared by reaction of a compound of the general formula:

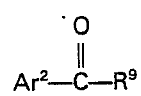

(VI)

with a compound of the general formula:

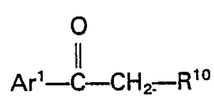

(VII)

where one of $Ar^1$ and $Ar^2$ represents the group Ar and the other represents the group:

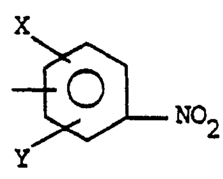

This reaction is a form of the Aldol condensation followed by a dehydration, and is carried out under the conditions usual for such reactions.

A —CO— group A or B in a compound of formula II or V may be derivatised by methods analogous to known methods to produce the other possible groups A or B which are, of course, alcohols, thiols, (thio)ethers, (thio)esters, (thio)acetals, oximes, oxime ethers, oxime esters, hydrazones and semicarbazones derived from the basic ketone group. Typical methods include, for example:

i) reaction to the ketone with the appropriate alcohol or glycol or thio-analogue thereof, to produce the desired acetal derivative. Such reactions are generally carried out under acidic conditions;

ii) reduction of the ketone using a suitable reducing agent to produce the corresponding alcohol. Typical reducing agents include hydrides, for example complex metal hydrides, for example sodium borohydride, or lithium aluminium hydride;

iii) reaction of the ketone with a Grignard reagent $R^3$Mg Hal where Hal is a halogen atom, especially a

bromine atom, and $R^3$ is an alkyl group, to produce the corresponding alcohol containing the group $—CR^3(OH)—$;

iv) reaction of an alcohol with hydrogen sulphide in the presence of an acidic catalyst, to produce the corresponding thiol;

v) alkylation of an alcohol or thiol, using a suitable alkylating agent, for example an alkyl halide, or an alcohol or thiol in the presence of an acidic catalyst, to produce the corresponding alkyl ether or thioether, and reactions of the alcohol or thiol with similar reagants to produce other ethers;

vi) esterification of an alcohol or thiol, using any suitable esterifying agent, to produce the corresponding carboxylic, sulphonic or phosphoric acid ester. Acyl halides, acid anhydrides and the acids themselves are suitable esterifying agents.

vii) reaction of the ketone with an ammonia derivative $H_2N—R^8$, under acidic conditions generally, to produce the corresponding $—(CN—R^8)—$ group. For example: the oxime $—(CN—OH)—$ may be prepared by reaction with hydroxylamine, the phenylhydrazone $—(CN—NHC_6H_5)—$ may be prepared from phenylhydrazine, the semicarbazone may be prepared from semicarbazide ($R=—NHCONH_2$), and so on.

A $—CR^9=CR^{10}—$ group A or B may be converted into a group

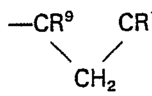 or 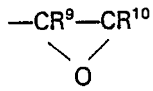

be reaction with any suitable nucleophilic methylene transfer reagent or epoxidising agent, or into a group $—CHR^9—CHR^{10}$ by reduction using any suitable reducing agent, for example hydrogen over a metallic catalyst. Alkyl groups $R^{11}$ and/or $R^{12}$ can be introduced by the use of suitable selective alkylating agents. For example, reaction of a compound in which one of A and B is a —CO— group and the other is a $—CR^9=CR^{10}—$ group with a cuprate alkylating agent such as $(R^{11})_2CuLi$ where $R^{11}$ is the desired alkyl group, produces a selectively alkylated product.

As stated above, a resulting compound of the general formula I may be converted into any other required compound of the general formula I. For example, the compound may be alkylated by reaction with an alkylating agent, for example an alkyl halide, especially an alkyl iodide, to produce a compound of the general formula I in which $R^1$ represents an alkyl group having 1 to 4 carbon atoms. Further, groups A and B in the resulting urea can be converted into other groups A and B by methods analogous to those described above for preparation of the intermediate compounds II or V. Thus for example if it is desired to produce a compound of the general formula I in which A represents a —CH(OH)— group, this can be done by reduction of a keto group A either in a compound of the general formula I or in a precursor compound II or V.

The compounds of the general formula exhibit herbicidal properties, and in some cases fungicidal properties. The invention therefore also provides a herbicidal and/or fungicidal composition which comprises a compound of the general formula I together with a carrier. The invention further provides a method of combating fungi and/or undesired plant growth at a locus, which comprises applying to the locus a compound of the general formula

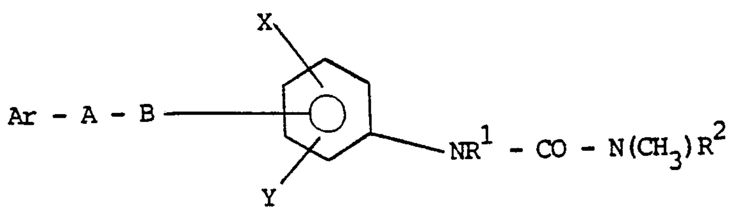 (I)

in which $R^1$ represents a hydrogen atom or an alkyl group having 1 to 4 carbon atoms; $R^2$ represents a hydrogen atom, a methoxy or a methyl group; Ar represents an optionally substituted phenyl group or an optionally substituted heteroaromatic ring containing 1 or 2 oxygen, sulphur and/or nitrogen atoms, optional substituents being selected from halogen atoms and alkyl, alkoxy, haloalkyl, haloalkoxy, alkylthio, phenyl, phenoxy, cyano, amino, alkylamino, amido, hydroxy, carboxy and alkoxycarbonyl groups, alkyl moieties in such substituents having up to 4 carbon atoms; each of X and Y independently represents a hydrogen or halogen atom or an alkyl, haloalkyl or alkoxy group; and one of A and B represents a group —CO—; $—CR^3(OR^4)—$ or $—CR^3(SR^4)—$, in which $R^3$ represents a hydrogen atom or an alkyl group and $R^4$ represents a hydrogen atom, an alkyl, alkenyl, alkynyl or cycloalkyl group, an optionally substituted phenyl or benzyl group, optional substituents being selected from halogen atoms and alkyl, alkoxy, haloalkyl, haloalkoxy, alkylthio, phenyl, phenoxy, cyano, amino, alkylamino, amido, hydroxy, carboxy and alkoxycarbonyl group, alkyl moieties in such substituents having up to 4 carbon atoms; or an acyl group derived from a carboxylic, carbamic, sulphonic or phosphoric acid; $C(OR^5)(OR^6)—$, $—C(OR^5)(SR^6—)$ or $—C(SR^5)(SR^6)—$, in which each of $R^5$ and $R^6$ independently represents an alkyl group or together represent an alkylene group; $—CH(CH_2COOR^7)—$ in which $R^7$ represents an alkyl group; or $—(CNR^8)—$ in which $R^8$ represents a group $OR^4$ or $HNR^4$; and the other of A and B represents a group $—CR^9R^{11}—CR^{10}R^{12}—$, in

which each of $R^9$, $R^{10}$, $R^{11}$ and $R^{12}$ independently represents a hydrogen atom or an alkyl group, or $R^{11}$ and $R^{12}$ together represents a carbon- carbon bond, a $—CH_2—$ group or an oxygen atom; but, in respect of designated states common to EP—A—81206 and the present application, and in relation to a method of combating undesired plant growth, is not a compound of formula I wherein $R^2$ represents a hydrogen atom or a methoxy group, or wherein $R^2$ represents a methyl group, $R^1$, X and Y are all hydrogen atoms, Ar is unsubstituted phenyl and the linkage —A—B— is $—CO—CH(CH_3)—CH_2—$or $(CH_2)_2—CO—$. The locus may for example be a crop area, containing, for example, plants or seeds of crops such as cereals or soya beans. The dosage of active ingredient used may for example be from 0.05 to 4 kg/ha.

When using the compounds or compositions of the invention as herbicides, treatment is preferably post-emergence. Treatment as a fungicide may, for example, be to plants, seeds or soil, as appropriate.

A carrier in a composition according to the invention is any material with which the active ingredient is formulated to facilitate application to the locus to be treated, or to facilitate storage, transport or handling. A carrier may be a solid or a liquid, including a material which is normally gaseous but which has been compressed to form a liquid, and any of the carriers normally used in formulating agricultural compositions may be used. Preferably compositions according to the invention contain 0.5 to 95% by weight of active ingredient.

Suitable solid carriers include natural and synthetic clays and silicates, for example natural silicas such as diatomaceous earths; magnesium silicate, for example talcs; magnesium aluminium silicates, for example attapulgites and vermiculites; aluminium silicates, for example kaolinites, montomorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; and chlorinated hydrocarbons, for example carbon tetrachloride perchloroethylene and trichloroethane. Mixtures of different liquids are often suitable.

Agricultural compositions are often formulated and transported in a concentrated form which is subsequently diluted by the user before application. The presence of small amounts of carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, or sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecylbenzene sulphonate; and polymers of etheylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3—10% of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing ½—10% w of active ingredient. Granules are usually prepared to have a size between 10 and 100 BS mesh (1.676—0.152 mm), and may be manufactured by agglomeration or impregnation techniques. Generally, granules will contain ½—75% w active ingredient and 0—10% w of additives such as stabilisers, surfactants, slow release modifiers and binding agents. The so-called "dry-flowable powders" consist of relatively small granules having a relatively high concentration of active ingredient. Emulsifiable concentrates usually contain, in addition to a solvent and, when necessary, co-solvent, 10—50% w/v active ingredient, 2—20% w/v emulsifiers and 0—20% w/v of other additives such as stabilisers, penetrants and corrosion inhibitors. Suspension concentrates are usually compounded so as to obtain a stable, non-sedimenting flowable product and usually contain 10—75% w active ingredient, 0.5—15% w of dispersing agents, 0.1—10% w of suspending agents such as protective colloids and thixotropic agents, 0—10% w of other additives such as defoamers, corrosion inhibitors, stabilisers, penetrants and stickers, and water or an organic liquid in which the ingredient is substantially insoluble; certain organic solids or inorganic salts may be present dissolved in the formulation to assist in preventing sedimentation or as anti-freeze agents

for water.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'-like consistency.

The compositions of the invention may also contain other ingredients, for example, other compounds possessing herbicidal, insecticidal or fungicidal properties.

The following Examples illustrate the invention. The identity of the products was confirmed by NMR — and mass spectral analysis as necessary, and where possible melting points were taken.

Example 1

Preparation of 1-(3-nitrophenyl)-3-(4-chlorophenyl)-prop-1-en-3-one

A solution of 3-nitrobenzaldehyde (13.7 g, 0.1 mol) and 4-chloroacetophenone (15.45 g, 0.1 mol) in absolute ethanol was cooled in an ice bath, then saturated with HCl gas and left to stand overnight. The crystalline product was then filtered off and recrystallized from acetone. 21 g of the desired product, corresponding to a yield of 73%, were obtained, the melting point being 111—112°C.

Example 2

Preparation of 2-(4-chlorophenyl)-2-[2-(3-nitrophenyl)ethynyl]-1,3-dioxolan

A mixture of the compound of Example 1 (5.8 g, 0.02 mol), ethylene glycol (20 ml) and *p*-toluene sulphonic acid (0.6 g) in toluene (300 ml) was refluxed overnight, removing water with a Dean-Stark trap. After cooling, the solution was washed with saturated sodium bicarbonate, then with water, then dried and evaporated. The product (6.5 g, 97% yield) was used for subsequent reactions without further purification.

Example 3

Preparation of 1-(3-nitrophenyl)-3-(4-chlorophenyl)-3-hydroxyprop-1-ene

To an ice cooled solution of the compound of Example 1 (8 g, 0.028 mol) in tetrahydrofuran (200 ml) was added a solution of $CeCl_3$ (6.8 g) in water (10 ml). The solution was diluted with methanol (75 ml) then treated slowly with sodium borohydride (1.06 g, 0.028 mol). After 1 hour the mixture was acidified with acetic acid, evaporated to low bulk, diluted with methylene chloride, washed with saturated $NaHCO_3$ and water, then dried and evaporated. Flash chromatography provided the allylic alcohol, 7.25 g (90% yield).

Example 4

Preparation of 1-(3-nitrophenyl)-3-(4-chlorophenyl)-3-methoxyprop-1-ene

A mixture of the compound of Example 3 (5 g, 0.017 mol) silver oxide (4.4 g) and methyl iodide (20 ml) was stirred overnight. The mixture was then filtered and the filtrate evaporated. After chromatography, 4.5 g (86%) of the methyl ester was obtained.

Example 5

Preparation of 1-(3-nitrophenyl)-2-(4-chlorobenzoyl)-cyclopropane

A mixture of NaH (0.9 g of a 50% dispersion, 0.18 mol) and trimethyl sulphoxonium iodide (4.3 g 0.018 mol) in dimethylsulphoxide (25 ml) at 0°C, under nitrogen, was stirred until $H_2$ evolution had ceased. A solution of the compound of Example 1 (5 g, 0.017 mol) in 50% tetrahydrofuran/dimethylsulphoxide (20 ml) was then added and after 5 minutes the ice bath was removed followed by a further 2 hours of stirring. The mixture was then poured into water, extracted with diethyl ether and the organic extracts washed several times with water, dried and evaporated. Chromatography provided 3.8 g (76% yield) of the cyclopropane.

Example 6

Preparation of 1-(3-aminophenyl)-3-(4-chlorophenyl)-prop-1-en-3-one

To a stirred suspension of the compound prepared in Example 1 (2.9 g, 0.01 mol) and 5% palladium on charcoal catalyst (0.3 g) in ethanol (200 ml), at room temperature, was added a solution of 10% aqueous sodium hypophosphite (50 ml). The mixture was then stirred until no starting material remained, as indicated by thin layer chromatography (about two hours). The mixture was then filtered through Celite (Trade Mark) and diluted with methylene chloride until the organic and aqueous layers separated. After separation the organic layer was washed with water, dried and evaporated, leaving the desired compound which was then purified by flash chromatography. 2.3 g of product, corresponding to a yield of 89%, were obtained.

Example 7

Preparation of 1-(3-aminophenyl)-3-(4-chlorophenyl)-propan-3-one

A suspension of the compound of Example 1 (2.9 g, 0.01 mol) and 5% platinum on charcoal (0.3 g), in ethanol (150 ml) was hydrogenated on a Parr hydrogenation apparatus until uptake of hydrogen had ceased. The mixture was then filtered through Celite (Trade Mark) and the filtrate evaporated, leaving the desired product in approximately quantitive yield.

Examples 8 to 22

By methods analogous to those of Examples 6 and 7, further compounds of the general formula II were prepared. Details are given in Table I.

TABLE I

| Example No | In the general formula II Ar | A | B | X | Y | Position of B relative to $NH_2$ |
|---|---|---|---|---|---|---|
| 8 | 4-chlorophenyl | 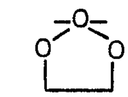 | —CH=CH— | H | H | *m* |
| 9 | phenyl | —CH(OH)— | —CH=CH— | H | H | *m* |
| 10 | phenyl | $—CH(OCH_3)—$ | —CH=CH— | H | H | *m* |
| 11 | phenyl | 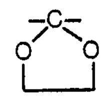 | —CH=CH— | H | H | *m* |
| 12 | 4-chlorophenyl | 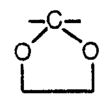 | $—CH_2CH_2—$ | H | H | *m* |
| 13 | 4-chlorophenyl | —CO— | $—CH_2CH_2—$ | H | H | *p* |
| 14 | 4-aminophenyl | —CO— | $—CH_2CH_2—$ | H | H | *m* |
| 15 | 4-chlorophenyl | $—CH(OCH_3)—$ | $—CH_2CH_2—$ | H | H | *m* |
| 16 | 3,4-dichlorophenyl | $—CH(OCH_3)—$ | $—CH_2CH_2—$ | H | H | *m* |
| 17 | 4-chlorophenyl | —CO— | 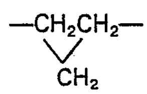 | H | H | *m* |
| 18 | 3,4-dichlorophenyl | 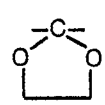 | $—CH_2CH_2—$ | H | H | *m* |
| 19 | phenyl | $—CH(OCH_3)—$ | $—CH_2CH_2—$ | H | H | *m* |
| 20 | phenyl | —CO— | $—CH_2CH_2—$ | H | H | *m* |
| 21 | phenyl | 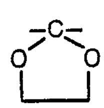 | $—CH_2CH_2—$ | H | H | *m* |
| 22 | 4-methyl-phenyl | —CO— | $—CH_2CH_2—$ | H | H | *m* |

Example 23

Preparation of 1-(3-[3-(4-chlorophenyl)-3-oxo-prop-1-enyl])-3,3-dimethylurea

The compound of Example 6 (2.0 g, 0.006 mol) was added to a saturated solution of phosgene in toluene (100 ml). After refluxing for 30 minutes nitrogen gas was bubbled through the mixture until all excess phosgene had been removed. The solution was then cooled to room temperature and treated with dimethylamine (2 ml). After 30 minutes the solvents were evaporated and the urea product crystallised from ethyl acetate. 2.0 g of product, corresponding to a yield of 93%, were obtained, melting point 159—160°C.

Elemental Analysis

| | C | H | N |
|---|---|---|---|
| Calculated | 64.6 | 5.6 | 7.5 |
| Found | 64.5 | 5.6 | 7.5 |

Example 24

Preparation of 1-(3-[3-(4-chlorophenyl)-3-oxopropyl]phenyl)-3,3-dimethylurea

By a method analogous to that described in Example 23, this compound was prepared from the compound of Example No. 7, molecular weight (mass spectrometer): 330.5.

Elemental Analysis

| | C | H | N |
|---|---|---|---|
| Calculated | 65.4 | 5.7 | 8.5 |
| Found | 65.7 | 5.7 | 8.7 |

Example 25

Preparation of 1-(3-[3-(4-chlorophenyl)-3-hydroxyphenyl]phenyl)-3,3-dimethylurea

An ice cooled solution of the compound of Example 24 (6.0 g, 0.018 mol) in methanol (200 ml) was treated with sodium borohydride (0.66 g, 0.018 mol) and stirred for 1 hour. The solution was then acidified with acetic acid, evaporated to low bulk, diluted with methylene chloride (200 ml) and washed first with saturated sodium bicarbonate (3 × 100 ml) then with water. After drying with sodium sulphate the solution was evaporated and the product chromotographed to provide 4.6 g (77% yield) of the desired alcohol as a thick oil, mol. weight 332.5.

Example 26

Preparation of 1-(3-[3-(4-chlorophenyl)-3-acetoxypropyl]phenyl)-3,3-dimethylurea

A solution of the compound of Example 25 (1.5 g, 0.005 mol) acetic anhydride (1 ml) and triethylamine (1 ml) in methylene chloride (50 ml) was refluxed for 5 hours. The solvents were then evaporated and the residue chromatographed to provide 0.9 g (48% yield) of the desired acetate as a thick oil. mo.l. weight 374.5.

Example 27

Preparation of 1-methyl-1-(3-[3-(4-chlorophenyl)-3-methoxypropyl]phenyl)-3,3-dimethylurea

A solution of the compound of Example 25 (1.66 g, 0.005 mol) in tetrahydrofuran (60 ml) was cooled in an ice bath before adding NaH (50% dispersion, 0.56 g). After stirring for 5 minutes methyl iodide (0.73 g) was added and the solution allowed to warm up to room temperature. Stirring was continued for 3 hours, then the reaction was quenched with 10% aqueous HCl, the product extracted into diethyl ether, washed with saturated sodium bicarbonate and water, then dried and evaporated. The product was purified by chromatography, to yield 1.53 g (55% yield) of the desired product, m.p. 121—2°C.

Example 28

Preparation of 1-(3-[3-(4-chlorophenyl)-3-methylsulphonyloxypropyl)-3,3-dimethylurea

A solution of the compound of Example 25 (1.5 g, 0.0047 mol) mesyl chloride (1 ml) and triethylamime (1 ml) in methylene chloride (50 ml) was refluxed for 1 hour. The solvents were then evaporated and the residue chromatographed to provide 0.9 g (47% yield) of the desired methylsulphonate, mol. weight 410.5.

Examples 29 to 97

By methods analogous those described in Examples 23 to 28, further compounds of the general formula I were prepared. Details are given in Table II.

TABLE II

| Example No | Ar | A | B | X Y $R^1$ $R^2$ | Position of B relative to —$NR^1$— | M. pt (°C) | M.w. |
|---|---|---|---|---|---|---|---|
| | | In the general Formula I | | | | | |
| 29 | 4-chlorophenyl | —CO— | —$CH_2CH_2$— | H H H $CH_3$ | *p* | 161—2 | |
| 30 | 4-chlorophenyl | 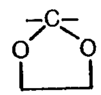 | —CH=CH— | H H H $CH_3$ | *m* | 159—60 | |
| 31 | 4-chlorophenyl | 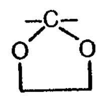 | —$CH_2CH_2$— | H H H $CH_3$ | *m* | 124—6 | |
| 32 | 4-chlorophenyl | —$CH(OCH_3)$— | —$CH_2CH_2$— | H H H $CH_3$ | *m* | 118—9 | |
| 33 | 4-chlorophenyl | —CO— | 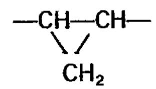 | H H H $CH_3$ | *m* | 152—3 | |
| 34 | 3,4-dichlorophenyl | 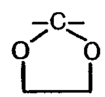 | —$CH_2CH_2$— | H H H $CH_3$ | *m* | 117—8 | |
| 35 | 3,4-dichlorophenyl | —$CH(OCH_3)$— | —$CH_2CH_2$— | H H H $CH_3$ | *m* | 124—5 | |
| 36 | phenyl | —$CH(OCH_3)$— | —$CH_2CH_2$— | H H $CH_3$ $CH_3$ | *m* | Oil | 326 |
| 37 | phenyl | —CH(OH)— | —$CH_2CH_2$— | H H H $CH_3$ | *m* | Oil | 298 |
| 38 | phenyl | —CH(OH)— | —CH=CH— | H H H $CH_3$ | *m* | — | 296 |
| 39 | phenyl | —CO— | —$CH_2CH_2$— | H H H $CH_3$ | *m* | 128—30 | |
| 40 | phenyl | —$CH(OCH_3)$— | —$CH_2CH_2$— | H H H $CH_3$ | *m* | 88—9 | |
| 41 | phenyl | —$CH(OCH_3)$— | —CH=CH— | H H H $CH_3$ | *m* | 117—9 | |
| 42 | phenyl | 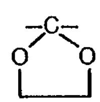 | —$CH_2CH_2$— | H H H $CH_3$ | *m* | 136—8 | |

TABLE II (cont)

| Example No | Ar | A (In the general Formula I) | B (In the general Formula I) | X Y $R^1$ $R^2$ (In the general Formula I) | Position of B relative to $—NR^1—$ | M. pt (°C) | M.w. |
|---|---|---|---|---|---|---|---|
| 43 | phenyl | 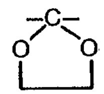 | $—CH{=}CH—$ | H H H $CH_3$ | *m* | 118—20 | |
| 44 | 4-chlorophenyl | $—CO—$ | $—CH{=}CH—$ | H H H $CH_3$ | *p* | 184—6 | |
| 45 | 4-methylphenyl | $—CH(OCH_3)—$ | $—CH_2CH_2—$ | H H H $CH_3$ | *m* | 99—101 | |
| 46 | 4-methylphenyl | $—CO—$ | $—CH{=}CH—$ | H H H $CH_3$ | *m* | 161—3 | |
| 47 | 4-methylphenyl | $—CO—$ | $—CH_2CH_2—$ | H H H $CH_3$ | *m* | 128—30 | |
| 48 | 4-methylphenyl | $—CH(OH)—$ | $—CH{=}CH—$ | H H H $CH_3$ | *m* | 105—7 | |
| 49 | 4-methylphenyl | $—CH(OH)—$ | $—CH_2CH_2—$ | H H H $CH_3$ | *m* | 95—7 | |
| 50 | phenyl | $—CH(OH)—$ | 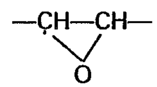 | H H H $CH_3$ | *m* | — | 312 |
| 51 | 4-methylphenyl | $—CH(OCH_3)—$ | $—CH_2CH_2—$ | H H $CH_3CH_3$ | *m* | Oil | 340 |
| 52 | 4-methoxyphenyl | $—CH(OCH_3)—$ | $—CH_2CH_2—$ | H H $CH_3CH_3$ | *m* | Oil | 356 |
| 53 | 4-methylphenyl | 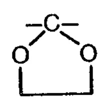 | $—CH{=}CH—$ | H H H $CH_3$ | *m* | 169—71 | |
| 54 | 4-methylphenyl | 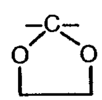 | $—CH_2CH_2—$ | H H H $CH_3$ | *m* | 85—7 | |
| 55 | 4-methoxyphenyl | $—CO—$ | $—CH_2CH_2—$ | H H H $CH_3$ | *m* | 137—9 | |
| 56 | 4-methoxyphenyl | $—CH(OH)—$ | $—CH_2CH_2—$ | H H H $CH_3$ | *m* | 89—90 | |
| 57 | 4-methoxyphenyl | $—CH(OCH_3)—$ | $—CH_2CH_2—$ | H H H $CH_3$ | *m* | 77—80 | |

TABLE II (cont)

| Example No | Ar | A | B | X Y $R^1$ $R^2$ | Position of B relative to $—NR^1—$ | M. pt (°C) | M.w. |
|---|---|---|---|---|---|---|---|
| | | In the general Formula I | | | | | |
| 58 | 4-methoxyphenyl | 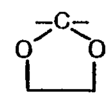 | $—CH_2CH_2—$ | H H H $CH_3$ | *m* | 104—6 | |
| 59 | 4-fluorophenyl | $—CH(OCH_3)—$ | $—CH_2CH_2—$ | H H H $CH_3$ | *m* | 89—91 | |
| 60 | 4-fluorophenyl | 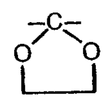 | $—CH=CH—$ | H H H $CH_3$ | *m* | 140—2 | |
| 61 | 4-fluorophenyl | 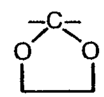 | $—CH_2CH_2—$ | H H H $CH_3$ | *m* | 142—4 | |
| 62 | 3-$CF_3$phenyl | $—CH(OCH_3)$ | $—CH_2CH_2—$ | H H H $CH_3$ | *m* | 95-6 | |
| 63 | 3-$CF_3$-phenyl | 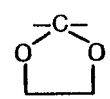 | $—CH_2CH_2—$ | H H H $CH_3$ | *m* | 133—4 | |
| 64 | 4-chlorophenyl | $—CH_2CH_2—$ | $—CH(OCH_3)—$ | H H H $CH_3$ | *m* | 82—4 | |
| 65 | 4-chlorophenyl | $—CH_2CH_2—$ | 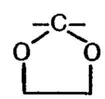 | H H H $CH_3$ | *m* | 123—5 | |
| 66 | 4-chlorophenyl | $—CH(OiC_3H_7)—$ | $—CH_2CH_2—$ | H H H $CH_3$ | *m* | Oil | 374.5 |
| 67 | 4-chlorophenyl | $—CH_2CH_2—$ | $—CH(OiC_3H_7)—$ | H H H $CH_3$ | *m* | | |
| 68 | 4-chlorophenyl | $—CH(OnC_4H_9)—$ | $-CH_2CH_2—$ | H H H $CH_3$ | *m* | Oil | 388.5 |
| 69 | 4-chlorophenyl | $—CH_2CH_2—$ | $—CH(OnC_4H_9)—$ | H H H $CH_3$ | *m* | | |
| 70 | 4-chlorophenyl | $—CH(OtC_4H_9)—$ | $—CH_2CH_2—$ | H H H $CH_3$ | *m* | Oil | 388.5 |
| 71 | 4-chlorophenyl | $—CH_2CH_2—$ | $—CH(OtC_4H_9—)$ | H H H $CH_3$ | *m* | | |

TABLE II (cont)

| Example No | Ar | A | B | X Y $R^1$ $R^2$ | Position of B relative to —$NR^1$— | M. pt (°C) | M.w. |
|---|---|---|---|---|---|---|---|
| | | In the general Formula I | | | | | |
| 72 | 4-chlorophenyl | —$CH(OcycloC_6H_{11})$— | —$CH_2CH_2$— | H H H $CH_3$ | *m* | Oil | 414.5 |
| 73 | 4-chlorophenyl | —$CH_2CH_2$— | —$CH(OcycloC_6H_{11})$— | H H H $CH_3$ | *m* | | |
| 74 | 4-chlorophenyl | —$CH(OCH_2C_6H_5)$— | —CH=CH— | H H H $CH_3$ | *m* | Oil | 420.5 |
| 75 | 4-chlorophenyl | —CH=CH— | —$CH(OCH_2C_6H_5)$— | H H H $CH_3$ | *m* | | |
| 76 | 4-chlorophenyl | —$CH_2$(2-ethylhexoxy)- | —$CH_2CH_2$— | H H H $CH_3$ | *m* | Oil | 444.5 |
| 77 | 4-chlorophenyl | —$CH_2CH_2$— | —CH(2-ethylhexoxy)— | H H H $CH_3$ | *m* | | |
| 78 | 4-chlorophenyl | —$CH(OC_2H_5)$— | —$CH_2$—$CH_2$— | H H H $CH_3$ | *m* | Oil | 360.5 |
| 79 | 4-chlorophenyl | —$CH_2CH_2$— | —$CH(OC_2H_5)$— | H H H $CH_3$ | *m* | | |
| 80 | 2-pyridyl | —$CH(OCH_3)$— | —$CH_2CH_2$ | H H H $CH_3$ | *m* | Oil | 313 |
| 81 | 4-chlorophenyl | —$CH_2CH_2$— | —$CH(OCH_3)$— | H *p*Cl H $CH_3$ | *m* | Oil | 381 |
| 82 | 4-chlorophenyl | —$CH(OCH_3)$— | —$CH_2CH_2$— | H *pCl H* $CH_3$ | *m* | | |
| 83 | 4-chlorophenyl | —CH=CH— | —$CH(SC_2H_5)$— | H H H $CH_3$ | *m* | Oil | 374 |
| 84 | 4-chlorophenyl | —$CH(SC_2H_5)$— | —CH=CH— | H H H $CH_3$ | *m* | | |
| 85 | 4-chlorophenyl | —CH=CH— | —$CH(SC_6H_5)$— | H H H $CH_3$ | *m* | 125—7 | |
| 86 | 4-chlorophenyl | —$CH(SC_6H_5)$- | —CH=CH— | H H H $CH_3$ | *m* | | |
| 87 | 4-chlorophenyl | —CH=CH— | —$CH(SCH_3)$— | H H H $CH_3$ | *m* | 124—6 | |
| 88 | 4-chlorophenyl | —$CH(SCH_3)$— | —CH=CH— | H H H $CH_3$ | *m* | | |

TABLE II (cont)

In the general Formula I

| Example No | Ar | A | B | X Y $R^1$ $R^2$ | Position of B relative to $-NR^1-$ | M. pt (°C) | M.w. |
|---|---|---|---|---|---|---|---|
| 89 | 4-methylphenyl | $-CH{=}CH-$ | $-CH(SC_2H_5)-$ | H H H $CH_3$ | *m* | 110—2 | |
| 90 | 4-methylphenyl | $-CH(SC_2H_5)-$ | $-CH{=}CH-$ | H H H $CH_3$ | *m* | | |
| 91 | phenyl | 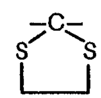 | $-CH{=}CH-$ | H H H $CH_3$ | *m* | 156—7 | |
| 92 | 4-chlorophenyl | $-C(NOH)-$ | $-CH_2CH_2-$ | H H H $CH_3$ | *m* | 184-6 | |
| 93 | 4-chlorophenyl | $-C(NOCH_3)-$ | $-CH_2CH_2-$ | H H H $CH_3$ | *m* | 142—4 | |
| 94 | 4-chlorophenyl | $-C(NOCOOCH_3)-$ | $-CH_2CH_2-$ | H H H $CH_3$ | *m* | 113—5 | |
| 95 | 4-chlorophenyl | —(NNHtosyl)— | $-CH_2CH_2-$ | H H H $CH_3$ | *m* | 83—5 | |
| 96 | 4-chlorophenyl | $-CH_2CH_2-$ | $-CH(CH_2COOC_2H_5)-$ | H H H $CH_3$ | *m* | Oil | 402 |

Example 97

Herbical Activity

To evaluate their herbicidal activity, compounds according to the invention were tested using as a representative range of plants: maize, *Zea mays* (Mz); rice, *Oryza sativa* (R); barnyard grass, *Echinochloa crusgalli* (BG); oat, *Avena sativa* (O); linseed, *Linum usitatissisum (L); mustard, Sinapsis alba* (M); sugar beet, *Beta vulgaris* (SB) and soya bean, *Glycine max* (S).

The tests fall into two categories, pre-emergence and post-emergence. The pre-emergence tests involved spraying a liquid formulation of the compound onto the soil in which the seeds of the plant species mentioned above had recently been sown. The post emergence tests involved two types of test, viz., soil drench and foliar spray tests. In the soil drench tests the soil in which the seedling plants of the above species were growing was drenched with a liquid formulation containing a compound of the invention, and in the foliar spray tests the seedling plants were sprayed with such a formulation.

The soil used in the tests was a prepared horticultural loam.

The formulations used in the tests were prepared by diluting with water, solutions of the test compounds in acetone containing 0.4% by weight of an alkylphenol/ethylene oxide condensate available under the trade mark TRITON X-155. The acetone solutions were diluted with water and the resulting formulations applied at dosage levels corresponding to 5 kg and/or 1 kg of active material per hectare in a volume equivalent to 600 liters per hectare in the soil spray and foliar spray tests, and at a dosage level equivalent to 10 kilograms of active material per hectare in a volume eqivalent to approximately 3,000 litres per hectare in the soil drench tests.

In the post-emergence tests untreated sown soil and in the post-emergence tests untreated soil bearing seedling plants were used as controls.

The herbicidal effects of the test compounds were assessed visually twelve days after spraying the foliage and the soil, and thirteen days after drenching the soil and were recorded on a 0—9 scale. A rating 0 indicates growth as untreated control, a rating 9 indicates death, An increase of 1 unit on the linear scale approximates to a 10% increase in the level of effect.

The results if the tests are set out in Table III below.

TABLE III

| Compound of Example No | Soil Drench 10 kg/ha Mz | R | BG | O | L | M | SB | S | Dosage kg/ha | Foliar Spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 23 | 0 | 0 | 0 | 3 | 0 | 0 | 9 | 4 | 5 | 0 | 0 | 0 | 0 | 4 | 7 | 3 | 0 | 0 | 0 | 0 | 0 | 0 | 0 | 0 | 0 |
| | | | | | | | | | 1 | 0 | 0 | 0 | 0 | 3 | 6 | 0 | 0 | 0 | 0 | 0 | 0 | 0 | 0 | 0 | 0 |
| 24 | 0 | 0 | 3 | 2 | 2 | 3 | 8 | 2 | 5 | — | — | — | — | — | — | — | — | 0 | 0 | 0 | 0 | 0 | 3 | 9 | 0 |
| | | | | | | | | | 1 | 0 | 0 | 3 | 0 | 4 | 8 | 9 | 4 | 0 | 0 | 0 | 0 | 0 | 1 | 9 | 0 |
| 25 | 0 | 0 | 0 | 0 | 0 | 4 | 9 | 4 | 5 | 0 | 0 | 0 | 0 | 4 | 8 | 9 | 4 | 0 | 0 | 0 | 0 | 0 | 5 | 8 | 0 |
| | | | | | | | | | 1 | 0 | 0 | 0 | 0 | 4 | 7 | 9 | 3 | 0 | 0 | 0 | 0 | 0 | 0 | 7 | 0 |
| 26 | 0 | 0 | 0 | 0 | 0 | 3 | 9 | 3 | 5 | — | — | — | — | — | — | — | — | 0 | 0 | 0 | 0 | 0 | — | 9 | — |
| | | | | | | | | | 1 | 0 | 0 | 0 | 4 | 7 | 9 | 4 | 4 | 0 | 0 | 0 | 0 | 0 | — | 7 | — |
| 27 | 0 | 0 | 0 | 0 | 0 | 0 | 0 | 0 | 5 | 4 | 0 | 4 | 5 | 9 | 9 | 9 | 6 | 0 | 0 | 0 | 0 | 0 | — | 4 | — |
| | | | | | | | | | 1 | 3 | 0 | 4 | 5 | 8 | 7 | 8 | 4 | 0 | 0 | 0 | 0 | 0 | — | 0 | — |
| 28 | 0 | 0 | 0 | 0 | 0 | 3 | 9 | 2 | 5 | 0 | 0 | 0 | 0 | 7 | 9 | 9 | 4 | 0 | 0 | 0 | 0 | 0 | — | 9 | — |
| | | | | | | | | | 1 | 0 | 0 | 0 | 0 | 7 | 9 | 9 | 4 | 0 | 0 | 0 | 0 | 0 | — | 7 | — |
| 29 | 0 | 0 | 0 | 0 | 0 | 0 | 6 | 0 | 5 | 0 | 0 | 0 | 0 | 2 | 6 | 7 | 4 | 0 | 0 | 0 | 0 | 0 | 0 | 7 | 0 |
| | | | | | | | | | 1 | 0 | 0 | 0 | 0 | 2 | 6 | 6 | 0 | 0 | 0 | 0 | 0 | 0 | 0 | 6 | 0 |
| 30 | 0 | 0 | 0 | 2 | 3 | 4 | 9 | 0 | 5 | — | — | — | — | — | — | — | — | 0 | 0 | 0 | 0 | 2 | 8 | 9 | 0 |
| | | | | | | | | | 1 | 2 | 0 | 4 | 3 | 8 | 8 | 9 | 4 | 0 | 0 | 0 | 0 | 0 | 5 | 7 | 0 |
| 31 | 0 | 0 | 0 | 0 | 0 | 0 | 0 | 0 | 5 | — | — | — | — | — | — | — | — | 0 | 0 | 0 | 0 | 0 | 7 | 9 | 0 |
| | | | | | | | | | 1 | 0 | 0 | 2 | 0 | 8 | 9 | 9 | 4 | 0 | 0 | 0 | 0 | 0 | 0 | 2 | 0 |

TABLE III (Cont)

| Compound of Example No | Soil Drench 10 kg/ha | | | | | | | | Dosage kg/ha | Foliar Spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 32 | 0 | 0 | 0 | 0 | 0 | 3 | 4 | 3 | — | — | — | — | — | — | — | — | — | — | — | — | — | — | — | — | — |
| | | | | | | | | | 1 | 4 | 3 | 7 | 5 | 9 | 9 | 9 | 6 | 0 | 0 | 5 | 4 | 0 | 4 | 9 | 3 |
| 33 | 0 | 0 | 0 | 0 | 0 | 7 | 0 | 0 | — | — | — | — | — | — | — | — | — | — | — | — | — | — | — | — | — |
| | | | | | | | | | 1 | 0 | 0 | 5 | 3 | 6 | 7 | 9 | 5 | 0 | 0 | 0 | 0 | 0 | 0 | 7 | 0 |
| 34 | 0 | 0 | 0 | 0 | 0 | 3 | 3 | 0 | — | — | — | — | — | — | — | — | — | — | — | — | — | — | — | — | — |
| | | | | | | | | | 1 | 2 | 0 | 6 | 3 | 8 | 9 | 9 | 4 | 0 | 0 | 0 | 0 | 2 | 7 | 8 | 0 |
| 35 | 0 | 0 | 0 | 0 | 0 | 7 | 4 | 0 | — | — | — | — | — | — | — | — | — | — | — | — | — | — | — | — | — |
| | | | | | | | | | 1 | 2 | 0 | 7 | 5 | 8 | 9 | 9 | 7 | 0 | 0 | 0 | 0 | 4 | 7 | 9 | 0 |
| 36 | 0 | 0 | 0 | 0 | 0 | 0 | 0 | 0 | — | — | — | — | — | — | — | — | — | — | — | — | — | — | — | — | — |
| | | | | | | | | | 1 | 2 | 2 | 5 | 2 | 5 | 8 | 9 | 6 | 0 | 0 | 0 | 0 | 0 | 3 | 0 | 0 |
| 37 | — | — | — | — | — | — | — | — | — | — | — | — | — | — | — | — | — | — | — | — | — | — | — | — | — |
| | | | | | | | | | 1 | 0 | 0 | 5 | 6 | 3 | 9 | 5 | 4 | 0 | 0 | 0 | 0 | 0 | 0 | 0 | 0 |
| 38 | 0 | 6 | 0 | 7 | 8 | 9 | 9 | 7 | 5 | 0 | 0 | 0 | 0 | 3 | 9 | 9 | 4 | 0 | 2 | 3 | 4 | 0 | 5 | 8 | 0 |
| | | | | | | | | | 1 | 0 | 0 | 0 | 0 | 2 | 9 | 8 | 3 | 0 | 0 | 0 | 2 | 0 | 2 | 6 | 0 |
| 39 | 0 | 3 | 2 | 5 | 0 | 3 | 8 | 4 | — | — | — | — | — | — | — | — | — | — | — | — | — | — | — | — | — |
| | | | | | | | | | 1 | 0 | 2 | 5 | 2 | 3 | 8 | 8 | 4 | 0 | 0 | 0 | 0 | 0 | 0 | 0 | 0 |
| 40 | — | — | — | — | — | — | — | — | — | — | — | — | — | — | — | — | — | — | — | — | — | — | — | — | — |
| | | | | | | | | | 1 | 1 | 3 | 7 | 6 | 9 | 9 | 9 | 7 | 0 | 0 | 0 | 0 | 0 | 7 | 8 | 0 |

TABLE III (cont)

| Compound of Example No | Soil Drench 10 kg/ha | | | | | | | | Dosage kg/ha | Foliar Spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 41 | — | — | — | — | — | — | — | — | — | — | — | — | — | — | — | — | — | — | — | — | — | — | — | — | — |
| | | | | | | | | | 1 | 1 | 5 | 8 | 6 | 9 | 9 | 9 | 6 | 0 | 0 | 0 | 0 | 0 | 0 | 0 | 0 |
| 42 | 2 | 8 | 3 | 5 | 6 | 9 | 9 | 6 | 5 | 2 | 4 | 5 | 2 | 9 | 9 | 9 | 6 | 0 | 0 | 0 | 4 | 5 | 8 | 9 | 2 |
| | | | | | | | | | 1 | 0 | 4 | 2 | 0 | 8 | 9 | 6 | 6 | 0 | 0 | 0 | 0 | 0 | 4 | 0 | 0 |
| 43 | 0 | 8 | 2 | 6 | 5 | 8 | 8 | 5 | 5 | 0 | 0 | 0 | 0 | 7 | 9 | 6 | 4 | 0 | 0 | 0 | 0 | 0 | 0 | 0 | 0 |
| | | | | | | | | | 1 | 0 | 0 | 0 | 0 | 7 | 8 | 6 | 3 | 0 | 0 | 0 | 0 | 0 | 0 | 0 | 0 |
| 44 | 0 | 0 | 0 | 0 | 2 | 0 | 3 | 2 | 5 | 0 | 0 | 0 | 2 | 0 | 2 | 6 | 0 | 0 | 0 | 0 | 0 | 0 | 0 | 0 | 0 |
| | | | | | | | | | 1 | 0 | 0 | 0 | 0 | 0 | 2 | 2 | 0 | 0 | 0 | 0 | 0 | 0 | 0 | 0 | 0 |
| 45 | 0 | 0 | 5 | 2 | 0 | 7 | 9 | 1 | 5 | 2 | 1 | 8 | 0 | 9 | 9 | 9 | 6 | 0 | 0 | 0 | 0 | 0 | 8 | 9 | 1 |
| | | | | | | | | | 1 | 0 | 0 | 8 | 0 | 8 | 9 | 9 | 6 | 0 | 0 | 0 | 0 | 0 | 6 | 8 | 0 |
| 46 | 3 | 0 | 2 | 0 | 0 | 8 | 0 | 3 | 5 | 0 | 0 | 0 | 0 | 0 | 9 | 2 | 3 | 0 | 0 | 0 | 0 | 0 | 4 | 4 | 0 |
| | | | | | | | | | 1 | 0 | 0 | 0 | 0 | 0 | 9 | 2 | 0 | 0 | 0 | 0 | 0 | 0 | 0 | 0 | 0 |
| 47 | 0 | 2 | 4 | 0 | 0 | 9 | 7 | 2 | 5 | 0 | 0 | 5 | 0 | 2 | 9 | 9 | 6 | 0 | 0 | 0 | 0 | 0 | 6 | 8 | 0 |
| | | | | | | | | | 1 | 0 | 0 | 4 | 0 | 0 | 9 | 6 | 5 | 0 | 0 | 0 | 0 | 0 | 0 | 3 | 0 |
| 48 | 0 | 2 | 3 | 2 | 2 | 9 | 9 | 4 | 5 | 0 | 0 | 7 | 0 | 9 | 9 | 9 | 6 | 0 | 0 | 0 | 0 | 0 | 8 | 8 | 2 |
| | | | | | | | | | 1 | 0 | 0 | 4 | 0 | 8 | 9 | 9 | 5 | 0 | 0 | 0 | 0 | 0 | 2 | 0 | 0 |
| 49 | 0 | 0 | 0 | 0 | 0 | 9 | 9 | 3 | 5 | 0 | 0 | 5 | 0 | 5 | 9 | 9 | 7 | 0 | 0 | 0 | 0 | 0 | 7 | 9 | 0 |
| | | | | | | | | | 1 | 0 | 0 | 5 | 0 | 0 | 9 | 9 | 6 | 0 | 0 | 0 | 0 | 0 | 0 | 2 | 0 |
| 50 | 0 | 4 | 0 | 5 | 0 | 9 | 7 | 6 | 5 | 0 | 0 | 0 | 0 | 2 | 9 | 7 | 5 | 0 | 0 | 0 | 0 | 3 | 4 | 6 | 0 |
| | | | | | | | | | 1 | — | — | — | — | — | — | — | — | 0 | 0 | 0 | 0 | 0 | 0 | 2 | 0 |

TABLE III (cont)

| Compound of Example No | Soil Drench 10 kg/ha | | | | | | | | Dosage kg/ha | Foliar Spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 51 | 0 | 0 | 0 | 0 | 0 | 0 | 0 | 0 | — | — | — | — | — | — | — | — | — | — | — | — | — | — | — | — | — |
| | | | | | | | | | 1 | 0 | 0 | 5 | 0 | 0 | 9 | 9 | 4 | 0 | 0 | 0 | 0 | 0 | 0 | 0 | 0 |
| 52 | 0 | 0 | 0 | 0 | 0 | 2 | 4 | 0 | — | — | — | — | — | — | — | — | — | — | — | — | — | — | — | — | — |
| | | | | | | | | | 1 | 3 | 0 | 8 | 4 | 6 | 9 | 9 | 6 | 0 | 0 | 0 | 0 | 0 | 2 | 6 | 2 |
| 53 | 0 | 0 | 0 | 3 | 2 | 9 | 3 | 3 | 5 | 4 | 5 | 9 | 0 | 5 | 9 | 8 | 6 | 0 | 0 | 0 | 0 | 0 | 0 | 0 | 0 |
| | | | | | | | | | 1 | 0 | 0 | 2 | 0 | 0 | 9 | 3 | 2 | 0 | 0 | 0 | 0 | 0 | 0 | 0 | 0 |
| 54 | 0 | 8 | 6 | 3 | 4 | 9 | 9 | 5 | 5 | 4 | 5 | 9 | 5 | 6 | 9 | 9 | 6 | 0 | 0 | 0 | 0 | 0 | 7 | 9 | 0 |
| | | | | | | | | | 1 | 0 | 0 | 7 | 0 | 4 | 9 | 9 | 5 | 0 | 0 | 0 | 0 | 0 | 2 | 3 | 0 |
| 55 | 0 | 0 | 0 | 0 | 0 | 0 | 0 | 0 | 5 | 0 | 0 | 8 | 3 | 2 | 9 | 9 | 4 | 0 | 0 | 0 | 0 | 0 | 3 | 7 | 0 |
| | | | | | | | | | 1 | 0 | 0 | 6 | 0 | 0 | 9 | 9 | 4 | 0 | 0 | 0 | 0 | 0 | 0 | 2 | 0 |
| 56 | 0 | 2 | 3 | 4 | 3 | 9 | 9 | 5 | 5 | 0 | 3 | 8 | 0 | 3 | 9 | 9 | 5 | 0 | 0 | 0 | 0 | 0 | 3 | 7 | 0 |
| | | | | | | | | | 1 | 0 | 0 | 8 | 0 | 0 | 9 | 9 | 4 | 0 | 0 | 0 | 0 | 0 | 0 | 2 | 0 |
| 57 | 0 | 2 | 5 | 5 | 9 | 9 | 9 | 4 | 5 | 4 | 5 | 9 | 6 | 8 | 9 | 9 | 8 | 0 | 0 | 3 | 3 | 2 | 8 | 8 | 3 |
| | | | | | | | | | 1 | 3 | 3 | 9 | 6 | 8 | 9 | 9 | 7 | 0 | 0 | 2 | 2 | 0 | 6 | 8 | 2 |
| 58 | 0 | 0 | 0 | 0 | 0 | 0 | 0 | 0 | 5 | 0 | 0 | 8 | 3 | 4 | 9 | 9 | 5 | 0 | 0 | 0 | 0 | 0 | 0 | 0 | 0 |
| | | | | | | | | | 1 | 0 | 0 | 3 | 0 | 0 | 7 | 0 | 3 | 0 | 0 | 0 | 0 | 0 | 0 | 0 | 0 |
| 59 | 0 | 4 | 6 | 2 | 5 | 5 | 9 | 5 | 5 | 5 | 6 | 9 | 6 | 8 | 9 | 9 | 8 | 0 | 0 | 3 | 3 | 4 | 7 | 6 | 0 |
| | | | | | | | | | 1 | 3 | 5 | 8 | 5 | 8 | 9 | 9 | 8 | 0 | 0 | 0 | 0 | 0 | 6 | 5 | 0 |

TABLE III (cont)

| Compound of Example No | Soil Drench 10 kg/ha | | | | | | | | Dosage kg/ha | Foliar Spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 60 | 0 | 0 | 4 | 0 | 3 | 7 | 0 | 4 | 5 | 2 | 3 | 8 | 6 | 8 | 9 | 9 | 7 | 0 | 0 | 0 | 0 | 0 | 7 | 8 | 0 |
| | | | | | | | | | 1 | 1 | 2 | 8 | 6 | 6 | 9 | 9 | 5 | 0 | 0 | 0 | 0 | 0 | 4 | 4 | 0 |
| 61 | 0 | 0 | 4 | 2 | 3 | 5 | 3 | 2 | 5 | 3 | 4 | 8 | 6 | 7 | 9 | 9 | 7 | 0 | 0 | 0 | 0 | 0 | 7 | 8 | 0 |
| | | | | | | | | | 1 | 2 | 2 | 7 | 4 | 6 | 9 | 9 | 7 | 0 | 0 | 0 | 0 | 0 | 3 | 3 | 0 |
| 62 | 0 | 0 | 0 | 0 | 0 | 3 | 7 | 3 | 5 | 5 | 6 | 9 | 7 | 9 | 9 | 9 | 9 | 0 | 0 | 0 | 0 | 3 | 8 | 9 | 3 |
| | | | | | | | | | 1 | 2 | 4 | 8 | 7 | 8 | 9 | 9 | 7 | 0 | 0 | 0 | 0 | 0 | 6 | 7 | 0 |
| 63 | 0 | 0 | 0 | 0 | 0 | 0 | 3 | 0 | 5 | 2 | 3 | 9 | 6 | 8 | 9 | 9 | 8 | 0 | 0 | 0 | 0 | 2 | 8 | 8 | 0 |
| | | | | | | | | | 1 | 0 | 0 | 7 | 6 | 8 | 8 | 9 | 7 | 0 | 0 | 0 | 0 | 0 | 4 | 7 | 0 |
| 64 | 0 | 0 | 0 | 4 | 4 | 4 | 9 | 0 | 5 | 3 | 2 | 9 | 6 | 9 | 9 | 9 | 5 | 0 | 0 | 0 | 0 | 3 | 6 | 7 | 3 |
| | | | | | | | | | 1 | 2 | 0 | 8 | 5 | 8 | 9 | 9 | 3 | 0 | 0 | 0 | 0 | 0 | 4 | 5 | 0 |
| 65 | 0 | 0 | 0 | 0 | 0 | 0 | 0 | 0 | 5 | 2 | 0 | 8 | 4 | 7 | 9 | 9 | 4 | 0 | 0 | 0 | 0 | 2 | 5 | 5 | 0 |
| | | | | | | | | | 1 | 2 | 0 | 8 | 4 | 4 | 8 | 7 | 4 | 0 | 0 | 0 | 0 | 0 | 3 | 2 | 0 |
| 32+64 | 0 | 0 | 0 | 0 | 3 | 9 | 6 | 0 | 5 | 4 | 2 | 9 | 7 | 9 | 9 | 9 | 7 | 0 | 0 | 0 | 0 | 0 | 7 | 8 | 0 |
| | | | | | | | | | 1 | 2 | 0 | 8 | 5 | 8 | 9 | 9 | 6 | 0 | 0 | 0 | 0 | 0 | 5 | 7 | 0 |
| 66+67 | 0 | 0 | 0 | 0 | 0 | 0 | 0 | 0 | 5 | 2 | 0 | 8 | 6 | 7 | 8 | 9 | 4 | 0 | 0 | 0 | 0 | 0 | 0 | 0 | 0 |
| | | | | | | | | | 1 | 0 | 0 | 7 | 3 | 2 | 7 | 7 | 2 | 0 | 0 | 0 | 0 | 0 | 0 | 0 | 0 |
| 68+69 | 0 | 0 | 0 | 0 | 0 | 5 | 2 | 0 | 5 | 3 | 2 | 8 | 6 | 8 | 9 | 9 | 5 | 0 | 0 | 0 | 0 | 0 | 8 | 8 | 0 |
| | | | | | | | | | 1 | 3 | 0 | 8 | 3 | 6 | 9 | 9 | 4 | 0 | 0 | 0 | 0 | 0 | 5 | 6 | 0 |

TABLE III (cont)

| Compound of Example No | Soil Drench 10 kg/ha | | | | | | | | Dosage kg/ha | Foliar Spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 70+71 | 0 | 0 | 0 | 0 | 0 | 0 | 0 | 0 | 5 | 3 | 0 | 8 | 5 | 7 | 9 | 9 | 3 | 0 | 0 | 0 | 0 | 0 | 4 | 4 | 0 |
| | | | | | | | | | 1 | 2 | 0 | 7 | 3 | 4 | 7 | 3 | 2 | 0 | 0 | 0 | 0 | 0 | 0 | 0 | 0 |
| 72+73 | 0 | 0 | 0 | 0 | 0 | 0 | 0 | 0 | 5 | 3 | 0 | 8 | 4 | 5 | 9 | 9 | 4 | 0 | 0 | 0 | 0 | 0 | 5 | 6 | 0 |
| | | | | | | | | | 1 | 1 | 0 | 7 | 0 | 4 | 8 | 6 | 0 | 0 | 0 | 0 | 0 | 0 | 2 | 2 | 0 |
| 74+75 | 0 | 0 | 0 | 0 | 0 | 0 | 0 | 0 | 5 | 3 | 0 | 8 | 4 | 8 | 9 | 9 | 4 | 0 | 0 | 0 | 0 | 0 | 5 | 5 | 0 |
| | | | | | | | | | 1 | 2 | 0 | 7 | 0 | 4 | 8 | 8 | 1 | 0 | 0 | 0 | 0 | 0 | 2 | 1 | 0 |
| 76+77 | 0 | 0 | 0 | 0 | 0 | 0 | 0 | 0 | 5 | 5 | 0 | 8 | 6 | 5 | 9 | 9 | 3 | 0 | 0 | 0 | 0 | 0 | 3 | 0 | 0 |
| | | | | | | | | | 1 | 0 | 0 | 7 | 2 | 2 | 6 | 5 | 1 | 0 | 0 | 0 | 0 | 0 | 0 | 0 | 0 |
| 78+79 | 0 | 0 | 4 | 3 | 0 | 3 | 8 | 3 | 5 | 4 | 8 | 9 | 7 | 8 | 9 | 9 | 7 | 0 | 0 | 0 | 0 | 0 | 8 | 8 | 0 |
| | | | | | | | | | 1 | 3 | 3 | 8 | 7 | 8 | 9 | 9 | 7 | 0 | 0 | 0 | 0 | 0 | 5 | 8 | 0 |
| 80 | 5 | 5 | 4 | 7 | 7 | 8 | 8 | 7 | 5 | 2 | 3 | 7 | 6 | 7 | 9 | 9 | 7 | 0 | 0 | 0 | 0 | 0 | 5 | 5 | 0 |
| | | | | | | | | | 1 | 2 | 2 | 7 | 2 | 5 | 8 | 9 | 6 | 0 | 0 | 0 | 0 | 0 | 0 | 2 | 0 |
| 81+82 | 0 | 0 | 0 | 0 | 0 | 0 | 0 | 0 | — | — | — | — | — | — | — | — | — | — | — | — | — | — | — | — | |
| | | | | | | | | | 1 | 0 | 0 | 5 | 0 | 5 | 8 | 7 | 3 | 0 | 0 | 0 | 0 | 0 | 3 | 5 | 0 |
| 83+84 | 0 | 0 | 0 | 0 | 0 | 0 | 0 | 0 | — | — | — | — | — | — | — | — | — | — | — | — | — | — | — | — | — |
| | | | | | | | | | 1 | 0 | 0 | 0 | 0 | 5 | 8 | 7 | 2 | 0 | 0 | 0 | 0 | 0 | 0 | 0 | 0 |
| 85+86 | 0 | 0 | 0 | 0 | 0 | 0 | 0 | 0 | 5 | 0 | 0 | 7 | 3 | 6 | 9 | 9 | 3 | 0 | 0 | 0 | 0 | 0 | 4 | 0 | 0 |
| | | | | | | | | | 1 | 0 | 0 | 0 | 0 | 3 | 7 | 2 | 1 | 0 | 0 | 0 | 0 | 0 | 0 | 0 | 0 |

TABLE III (cont)

| Compound of Example No | Soil Drench 10 kg/ha | | | | | | | | Dosage kg/ha | Foliar Spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 87+88 | 0 | 0 | 0 | 0 | 0 | 0 | 0 | 0 | 5 | 0 | 0 | 7 | 3 | 8 | 9 | 9 | 4 | 0 | 0 | 4 | 2 | 0 | 7 | 9 | 5 |
| | | | | | | | | | 1 | 0 | 0 | 3 | 0 | 3 | 9 | 8 | 2 | 0 | 0 | 0 | 0 | 0 | 3 | 0 | 0 |
| 89+90 | 0 | 0 | 0 | 0 | 0 | 0 | 0 | 0 | 5 | 4 | 2 | 8 | 5 | 9 | 9 | 9 | 8 | 0 | 0 | 0 | 0 | 0 | 8 | 9 | 2 |
| | | | | | | | | | 1 | 4 | 2 | 7 | 5 | 9 | 9 | 9 | 5 | 0 | 0 | 0 | 0 | 0 | 2 | 2 | 2 |
| 91 | 0 | 0 | 0 | 0 | 0 | 0 | 0 | 0 | — | — | — | — | — | — | — | — | — | — | — | — | — | — | — | — | — |
| | | | | | | | | | 1 | 0 | 0 | 0 | 0 | 3 | 8 | 1 | 0 | 0 | 0 | 0 | 0 | 0 | 0 | 0 | 0 |
| 92 | 0 | 0 | 0 | 0 | 0 | 0 | 0 | 0 | 5 | 3 | 0 | 8 | 2 | 4 | 8 | 8 | 7 | 0 | 0 | 0 | 0 | 0 | 0 | 0 | 0 |
| | | | | | | | | | 1 | 2 | 0 | 7 | 2 | 0 | 7 | 8 | 6 | 0 | 0 | 0 | 0 | 0 | 0 | 0 | 0 |
| 93 | 0 | 0 | 0 | 0 | 0 | 0 | 0 | 0 | 5 | 2 | 3 | 8 | 6 | 7 | 9 | 9 | 5 | 0 | 0 | 0 | 0 | 0 | 0 | 0 | 0 |
| | | | | | | | | | 1 | 0 | 0 | 4 | 2 | 3 | 5 | 3 | 3 | 0 | 0 | 0 | 0 | 0 | 0 | 0 | 0 |
| 94 | 0 | 0 | 0 | 0 | 0 | 0 | 0 | 0 | 5 | 0 | 0 | 7 | 0 | 5 | 9 | 9 | 5 | 0 | 0 | 0 | 0 | 0 | 0 | 0 | 0 |
| | | | | | | | | | 1 | 0 | 0 | 0 | 0 | 2 | 5 | 5 | 3 | 0 | 0 | 0 | 0 | 0 | 0 | 0 | 0 |
| 95 | 2 | 1 | 0 | 0 | 1 | 0 | 9 | 0 | 5 | 4 | 2 | 7 | 2 | 8 | 9 | 9 | 7 | 0 | 0 | 0 | 0 | 0 | 0 | 0 | 0 |
| | | | | | | | | | 1 | 2 | 0 | 3 | 0 | 6 | 9 | 9 | 4 | 0 | 0 | 0 | 0 | 0 | 0 | 0 | 0 |
| 96 | 0 | 0 | 2 | 5 | 0 | 4 | 5 | 0 | — | — | — | — | — | — | — | — | — | — | — | — | — | — | — | — | |
| | | | | | | | | | 1 | 0 | 3 | 0 | 6 | 3 | 7 | 6 | 4 | 0 | 3 | 5 | 3 | 2 | 3 | 4 | 2 |

Example 98

Fungicidal Activity

(a) Activity against vine downy mildew (Plasmopara viticola; Pva

This test is a direct antisporulant one using a foliar spray. The lower surfaces of leaves of whole vine plants were inoculated by spraying with an aqueous suspension containing $10^5$ zoosporangia/ml 2 days prior to treatment with the test compound. The inoculated plants were kept for 24 hours in a high humidity compartment, and then 24 hours at glasshouse ambient temperature and humidity. The plants were then dried and infected leaves detached and sprayed on the lower surfaces with a solution of active material in 1:1 water/acetone containing 0.04% Triton X—155 (Trade Mark). The spraying was carried out with a moving track sprayer which delivered 620 l/ha, and the concentration of active material was calculated to give an application rate of 1kg/ha. After drying, the petioles of the sprayed leaves were dipped in water and the leaves returned to high humidity for a further 96 hours incubation, followed by assessment. Assessment was based on the percentage of the leaf area covered by sporulation compared with that on control leaves.

(b) Activity against vine downy mildew (Plasmopara viticola; Pvt.)

This test is a translaminar protectant one using a foliar spray. The upper surfaces of whole vine plants were sprayed at a dosage of 1 kilogram of active material per hectare using a track sprayer. The lower surfaces of the leaves were then inoculated, up to 6 hours after treatment with the test compounds, by spraying with an aqueous suspension containing $10^5$ zoosporangia/ml. The inoculated plants wre kept for 24 hours in a high humidity compartment, 4 days at glasshouse ambient temperature and humidity and then returned for a further 24 hours to high humidity. Assessment was based on the percentage of leaf area covered by sporulation compared with that on control leaves.

(c) Activity against barley powdery mildew (Erysiphe graminis; Eg.)

This test measures the direct antisporulant activity of compounds applied as a foliar spray. For each compound about 40 barley seedlings were grown to the one-leaf stage in a plastic pot of sterile potting compost. Inoculation was effected by dusting the leaves with conidia of Erysiphe graminis, spp. hordei. 24 hours after inoculation the seedlings were sprayed with a solution of the compound in a mixture of acetone (50%), surfactant (0.04%) and water using a track sprayer as described under (a). The rate of application was equivalent to 1kg of active material per hectare. First assessment of disease was made 5 days after treatment, when the overall level of sporulation on the treated plants was compared with that on control plants.

(d) Activity against apple powdery mildew (Podsophaera leucotrica; Pl)

This test is a direct anti-sporulant one using a foliar spray. The upper surfaces of whole leaves of whole apple seedlings were incoculated by spraying with an aqueous suspension containing $10^5$ conidia/ml 2 days prior to treatment with the test compound. The inoculated plants were immediately dried and kept at glasshouse ambient temperatures and humidity prior to treatment. The plants were sprayed at a dosage of 1 kilogram of active material per hectare using a track sprayer. After drying the plants were returned to a compartment at ambient temperature and humidity for up to 9 days, followed by assessment. Assessment was based on the percentage of the leaf area covered by sporulation compared with that on leaves of control plants.

(e) Activity against barley powdery mildew (Erysiphe graminis; Egs)

This test measures the systemic protectant activity of compounds applied as a soil drench. For each compound, soil was drenched at a dosage equivalent to 10kg/ha. About 25 barley seeds, cv. Golden Promise, were placed on top of the treated soil, and covered with about 1 cm untreated soil. After about 7 days when the seeds had germinated, spores of the fungus were dusted onto the plants. After 5 days, the level of sporulation was compared with that on control plants.

(f) Activity against broad bean rust (Uromyces fabae; Uf)

This test measures the translaminar antisporulant activity of compounds applied as a foliar spray. For each compound, seedling broad bean plants with two pairs of leaves were inoculated with an aqueous suspension containing $10^4$ spores/ml. After 24 hours, the plants were sprayed with the test compound at a rate equivalent to 1kg/ha. Assessment, after about 12 days, is by comparing the relative density of sporulating pustules compared with controls.

The extent of the disease control achieved in these tests is expressed as a control rating in Table IV below; greater than 80% disease control is given the rating 2 after the test; control of between 50% and 80% is given the rating 1 after the test.

TABLE IV

| Compound of Example No. | Tests in which Greater than 50% Disease Control Obtained |
|---|---|
| 23 | Pva (1) |
| 25 | Pva (1) |
| 26 | Uf (1) |
| 27 | Pva (2) |
| 32 | Eg (2); Pl (1) |
| 33 | Uf (1) |
| 34 | Pva (1); Eg (1) |
| 35 | Pva (2); Eg (1) |
| 37 | Pva (1) |
| 38 | Egs (1); Uf (1) |
| 39 | Uf (1) |
| 40 | Pva (1); Pvt (2); Egs (2) |
| 41 | Pva (2); Pvt (2); Eg (1) |
| 42 | Pva (1); Egs (1) |
| 43 | Pva (1); Uf (1) |
| 45 | Pva (1); Eg (2); Uf (1) |
| 47 | Uf (1) |
| 49 | Uf (1) |
| 50 | Uf (1) |
| 51 | Pva (1); Pvt (1); Uf (1) |
| 52 | Pva (1); Pvt (1) |
| 56 | Pva (1) |
| 57 | Pva (1) |
| 59 | Pvt (1); Eg (1); Pl (1); Egs (2) |
| 60 | Uf (1) |
| 61 | Uf (1) |
| 62 | Pvt (1); Eg (1) |
| 63 | Pvt (1); Eg (1); Uf (1) |
| 68+69 | Eg (1) |
| 78+79 | Pvt (1); Eg (1) |

TABLE IV (continued)

| Compound of Example No. | Tests in which Greater than 50% Disease Control Obtained |
|---|---|
| 80 | Uf (1) |
| 81+82 | Uf (1) |
| 91 | Uf (1) |
| 93 | Pva (1) |
| 95 | Pvt (1) |

**Claims for the Contracting States: BE CH DE FR GB IT LI NL**

1. A compound of the general formula

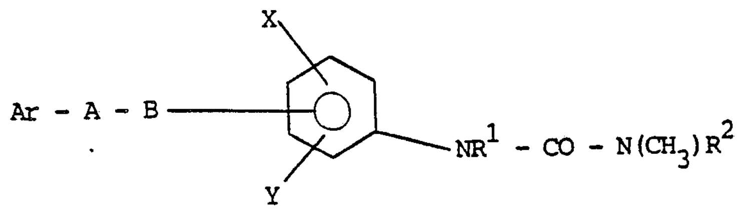

(I)

in which $R^1$ represents a hydrogen atom or an alkyl group having 1 to 4 carbon atoms; $R^2$ represents a methyl group; Ar represents an optionally substituted phenyl group or an optionally substituted heteroaromatic ring containing 1 or 2 oxygen, sulphur and/or nitrogen atoms, optional substituents being selected from halogen atoms and alkyl, alkoxy, haloalkyl, haloalkoxy, alkylthio, phenyl, phenoxy, cyano, amino, alkylamino, amido, hydroxy, carboxy and alkoxycarbonyl groups, alkyl moieties in such substituents having up to 4 carbon atoms; each of X and Y independently represents a hydrogen or halogen atom or an alkyl, haloalkyl or alkoxy group; and one of A and B represents a group —CO—; —$CR^3(OR^4)$— or —$CR^3(SR^4)$—, in which $R^3$ represents a hydrogen atom or an alkyl group and $R^4$ represents a hydrogen atom, an alkyl, alkenyl, alkynyl or cycloalkyl group, an optionally substituted phenyl or benzyl group, optional substituents being selected from halogen atoms and alkyl, alkoxy, haloalkyl, haloalkoxy, alkylthio, phenyl, phenoxy, cyano, amino, alkylamino, amido, hydroxy, carboxy and alkoxycarbonyl groups, alkyl moieties in such substituents having up to 4 carbon atoms; or an acyl group derived from a carboxylic, carbamic, sulphonic or phosphoric acid; $C(OR^5)(OR^6)$—, —$C(OR^5)(SR^6)$— or —$C(SR^5)(SR^6)$—, in which each of $R^5$ and $R^6$ independently represents an alkyl group or together represent an alkylene group; —$CH(CH_2COOR^7)$— in which $R^7$ represents an alkyl group; or —$(CNR^8)$— in which $R^8$ represents a group $OR^4$ or $HN^4$; and the other of A and B represents a group —$CR^9R^{11}$—$CR^{10}R^{12}$—, in which each of $R^9$, $R^{10}$, $R^{11}$ and $R^{12}$ independently represents a hydrogen atom or an alkyl group, or $R^{11}$ and $R^{12}$ together represent a carbon- carbon bond, a —$CH_2$— group or an oxygen atom; but not including compounds of formula I wherein $R^1$, X and Y are all hydrogen atoms, Ar is unsubstituted phenyl and the linkage —A—B— is —CO—$CH(CH_3)$—$CH_2$— or —$(CH_2)_2$—CO—.

2. A compound as claimed in claim 1, in which $R^1$ represents a methyl group or a hydrogen atom.

3. A compound as claimed in claims 1 or 2, in which Ar represents an unsubstituted pyridine ring or a phenyl group which is unsubstituted or substituted by one or more substituents selected from halogen atoms and $C_{1-4}$ alkyl, alkoxy and haloalkyl groups.

4. A compound as claimed in any one of claims 1 to 3, in which both X and Y represent hydrogen atoms.

5. A compound as claimed in any one of claims 1 to 4, in which one of A and B represents a group —CO—, —$CR^3(OR^4)$—, —$C(OR^5)(OR^6)$— or —$C(SR^5)(SR^6)$—.

6. A compound as claimed in any preceding claim, in which $R^3$ represents a hydrogen atom.

7. A compound as claimed in any preceding claim wherein $R^4$ represents a hydrogen atom or a $C_{1-4}$ alkyl, ($C_{1-4}$ alkyl)— carbonyl or methylsulphonyl group; and either each of $R^5$ and $R^6$ independently represents a $C_{1-4}$ alkyl group or $R^5$ and $R^6$ together represent a dimethylene or trimethylene group.

8. A compound as claimed in any preceding claim, in which one of A and B represents a group —CO—, —CH(OH)—, —$CH(OCH_3)$ or

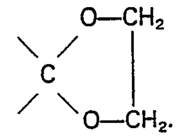

9. A compound as claimed in any one of claims 1 to 8, in which one of A and B represents a group $-CH=CH-$ or $-CH_2-CH_2-$.

10. A compound as claimed in claim 5, 6 or 7 and in claim 8, wherein A represents the group of formula $-CO-$, $-CR^3(OR^4)-$, $-C(OR^5)(OR^6)$ or $-C(SR^5)(SR^6)$ and B represents the group of formula $-CH=CH-$ or $-CH_2-CH_2$.

11. A process for the preparation of a compound as claimed in claim 1, which comprises reacting a compound of the general formula:

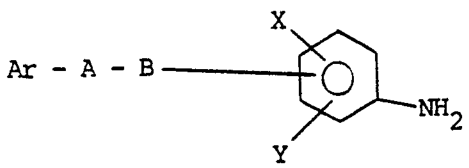

(II)

in which Ar, A, B, X and Y have the meanings given for the general formula I, with either

(a) a compound of the general formula:

$$Hal-CO-N(CH_3)R^2 \qquad (III)$$

in which $R^2$ has the meaning given for the general formula I and Hal represents a halogen atom; or

(b) phosgene, to produce the corresponding isocyanate, and reacting at least part of the isocyanate with an amine of the general formula:

$$HN(CH_3)R^2 \qquad (IV)$$

in which formula $R^2$ has the meaning given for the general formula I;
to produce a compound of the general formula I in which $R^1$ represents a hydrogen atom; and if desired, converting the resulting compound of the general formula I into any other desired compound of the general formula I.

12. A herbicidal and/or fungicidal composition which comprises a compound as claimed in any one of claims 1 to 10 together with a carrier.

13. A compound as claimed in claim 12, which comprises at least two carriers, at least one of which is a surface-active agent.

14. A method of combating fungi and/or undesired plant growth at a locus which comprises applying to the locus a command of the general formula

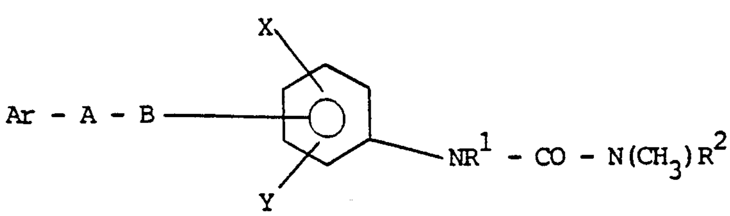

(I)

in which $R^1$ represents a hydrogen atom or an alkyl group having 1 to 4 carbon atoms; $R^2$ represents a methyl group; Ar represents an optionally substituted phenyl group or an optionally substituted heteroaromatic ring containing 1 or 2 oxygen, sulphur and/or nitrogen atoms, optional substituents being selected from halogen atoms and alkyl, alkoxy, haloalkyl, haloalkoxy, alkylthio, phenyl, phenoxy, cyano, amino, alkylamino, amido, hydroxy, carboxy and alkoxycarbonyl groups, alkyl moieties in such substituents having up to 4 carbon atoms; each of X and Y independently represents a hydrogen or halogen atom or an alkyl, haloalkyl or alkoxy group; and one of A and B represents a group $-CO-$; $-CR^3(OR^4)-$ or $-CR^3(SR^4)-$, in which $R^3$ represents a hydrogen atom or an alkyl group and $R^4$ represents a hydrogen atom, an alkyl, alkenyl, alkynyl or cycloalkyl group, an optionally substituted phenyl or benzyl group, optional substituents being selected from halogen atoms and alkyl, alkoxy, haloalkyl, haloalkoxy, alkylthio, phenyl, phenoxy, cyano, amino, alkylamino, amido, hydroxy, carboxy and alkoxycarbonyl groups, alkyl moieties in such substituents having up to 4 carbon atoms; or an acyl group derived from a carboxylic, carbamic, sulphonic or phosporic acid) $C(OR^5)(OR^6)-$, $-C(OR^5)(SR^6)-$ or $-C(SR^5)(SR^6)-$, in which each of $R^5$ and $R^6$ independently represents an alkyl group or together represent an alkylene group;

$—CH(CH_2COOR^7)—$ in which $R^7$ represents an alkyl group; or $—(CNR^8)—$ in which $R^8$ represents a group $OR^4$ or $HNR^4$; and the other of A and B represents a group $—CR^9R^{11}—CR^{10}R^{12}—$, in which each of $R^9$, $R^{10}$, $R^{11}$ and $R^{12}$ independently represents a hydrogen atom or an alkyl group, or $R^{11}$ and $R^{12}$ together represent a carbon- carbon bond, a $—CH_2—$ group or an oxygen atom; but, in a method of combating undesired plant growth, is not a compound of formula I wherein $R^1$, X and Y are all hydrogen atoms, Ar is unsubstituted phenyl and the linkage —A—B— is $—CO—CH(CH_3)—CH_2—$ or $—(CH_2)_2—CO—$.

**Claims for the Contracting State: AT**

1. A herbicidal and/or fungicidal composition which comprises an active ingredient together with a carrier, characterised in that the active ingredient is a compound of the general formula

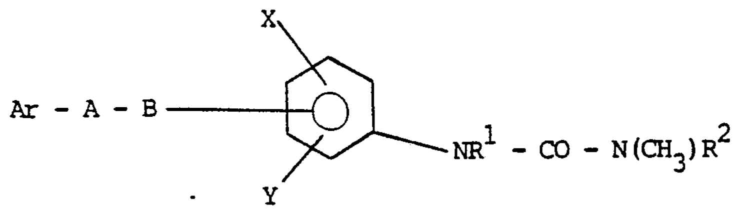

(I)

in which $R^1$ represents a hydrogen atom or an alkyl group having 1 to 4 carbon atoms; $R^2$ represents a methyl group; Ar represents an optionally substituted phenyl group or an optionally substituted heteroaromatic ring containing 1 or 2 oxygen, sulphur and/or nitrogen atoms, optional substituents being selected from halogen atoms and alkyl, alkoxy, haloalkyl, haloalkoxy, alkylthio, phenyl, phenoxy, cyano, amino, alkylamino, amido, hydroxy, carboxy and alkoxycarbonyl groups, alkyl moieties in such substituents having up to 4 carbon atoms; each of X and Y independently represents a hydrogen or halogen atom or an alkyl, haloalkyl or alkoxy group; and one of A and B represents a group —CO—; $—CR^3(OR^4)—$ or $—CR^3(SR^4)—$, in which $R^3$ represents a hydrogen atom or an alkyl group and $R^4$ represents a hydrogen atom, an alkyl, alkenyl, alkynyl or cycloalkyl group, an optionally substituted phenyl or benzyl group, optional substituents being selected from halogen atoms and alkyl, alkoxy, haloalkyl, haloalkoxy, alkylthio, phenyl, phenoxy, cyano, amino, alkylamino, amido, hydroxy, carboxy and alkoxycarbonyl groups, alkyl moieties in such substituents having up to 4 carbon atoms; or an acyl group derived from a carboxylic, carbamic, sulphonic or phosphoric acid; $C(OR^5)(OR^6)—$, $—C(OR^5)(SR^6)—$ or $—C(SR^5)(SR^6)—$, in which each of $R^5$ and $R^6$ independently represents an alkyl group or together represent an alkylene group; $—CH(CH_2COOR^7)—$ in which $R^7$ represents an alkyl group; or $—(CNR^8)—$ in which $R^8$ represents a group $OR^4$ or $HNR^4$; and the other of A and B represents a group $—CR^9R^{11}—CR^{10}R^{12}—$, in which each of $R^9$, $R^{10}$, $R^{11}$ and $R^{12}$ independently represents a hydrogen atom or an alkyl group, or $R^{11}$ and $R^{12}$ together represent a carbon- carbon bond, a $—CH_2—$ group or an oxygen atom; but not including compounds of formula I wherein $R^1$, X and Y are all hydrogen atoms, Ar is unsubstituted phenyl and the linkage —A—B— is $—CO—CH(CH_3)—CH_2—$ or $—(CH_2)_2—CO—$.

2. A composition as claimed in claim 1, in which $R^1$ represents a methyl group or a hydrogen atom.

3. A composition as claimed in claims 1 or 2, in which Ar represents an unsubstituted pyridine ring or a phenyl group which is unsubstituted or substituted by one or more substituents selected from halogen atoms and $C_{1-4}$ alkyl, alkoxy and haloalkyl groups.

4. A composition as claimed in any one of claims 1 to 3, in which both X and Y represent hydrogen atoms.

5. A composition as claimed in any one of claims 1 to 4, in which one of A and B represents a group —CO—, $—CR^3(OR^4)—$, $—C(OR^5)(OR^6)—$ or $—C(SR^5)(SR^6)—$.

6. A composition as claimed in any preceding claim, in which $R^3$ represents a hydrogen atom.

7. A composition as claimed in any preceding claim wherein $R^4$ represents a hydrogen atom or a $C_{1-4}$ alkyl, ($C_{1-4}$ alkyl)— carbonyl or methylsulphonyl group; and either each of $R^5$ and $R^6$ independently represents a $C_{1-4}$ alkyl group or $R^5$ and $R^6$ together represent a dimethylene or trimethylene group.

8. A composition as claimed in any preceding claim, in which one of A and B represents a group —CO—, —CH(OH)—, $—CH(OCH_3)$ or

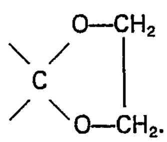

9. A composition as claimed in any one of claims 1 to 8, in which one of A and B represents a group —CH=CH— or $—CH_2—CH_2—$.

10. A composition as claimed in claim 5, 6 or 7 and in claim 8, wherein A represents the group of formula —CO—, $—CR^3(OR^4)—$, $—C(OR^5)(OR^6)$ or $—C(SR^5)(SR^6)$ and B represents the group of formula —CH=CH— or $—CH_2—CH_2$.

11. A composition as claimed in any preceding claim, which comprises at least two carriers, at least one of which is a surface-active agent.

12. A process for the preparation of formula I, as defined in claim 1, which comprises reacting a compound of the general formula:

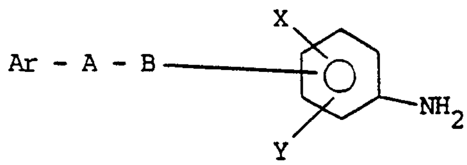

(II)

in which Ar, A, B, X and Y have the meanings given for the general formula I, with either

(a) a compound of the general formula:

$$Hal—CO—N(CH_3)R^2 \quad (III)$$

in which $R^2$ has the meaning given for the general formula I and Hal represents a halogen atom; or

(b) phosgene, to produce the corresponding isocyanate, and reacting at least part of the isocyanate with an amine of the general formula:

$$HN(CH_3)R^2 \quad (IV)$$

in which $R^2$ has the meaning given for the general formula I;

to produce a compound of the general formula I in which $R^1$ represents a hydrogen atom; and if desired, converting the resulting compound of the general formula I into any other desired compound of the general formula I.

13. A method of combating fungi and/or undesired plant growth at a locus which comprises applying to the locus a composition in which the active ingredient is a compound of the general formula

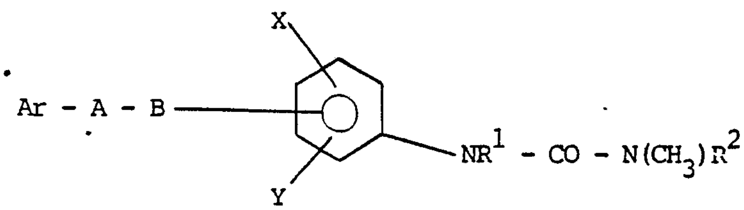

(I)

in which $R^1$ represents a hydrogen atom or an alkyl group having 1 to 4 carbon atoms; $R^2$ represents a methyl group; Ar represents an optionally substituted phenyl group or an optionally substituted heteroaromatic ring containing 1 or 2 oxygen, sulphur and/or nitrogen atoms, optional substituents being selected from halogen atoms and alkyl, alkoxy, haloalkyl, haloalkoxy, alkylthio, phenyl, phenoxy, cyano, amino, alkylamino, amido, hydroxy, carboxy and alkoxycarbonyl groups, alkyl moieties in such substituents having up to 4 carbon atoms; each of X and Y independently represents a hydrogen or halogen atom or an alkyl, haloalkyl or alkoxy group; and one of A and B represents a group —CO—; $—CR^3(OR^4)—$ or $—CR^3(SR^4)—$, in which $R^3$ represents a hydrogen atom or an alkyl group and $R^4$ represents a hydrogen atom, an alkyl, alkenyl, alkynyl or cycloalkyl group, an optionally substituted phenyl or benzyl group, optional substituents being selected from halogen atoms and alkyl, alkoxy, haloalkyl, haloalkoxy, alkylthio, phenyl, phenoxy, cyano, amino, alkylamino, amido, hydroxy, carboxy and alkoxycarbonyl groups, alkyl moieties in such substituents having up to 4 carbon atoms; or an acyl group derived from a carboxylic, carbamic, sulphonic or phosphoric acid; $C(OR^5(OR^6)—$, $—C(OR^5)(SR^6)—$ or $—C(SR^5)(SR^6)—$, in which each of $R^5$ and $R^6$ independently represents an alkyl group or together represent an alkylene group; $—CH(CH_2COOR^7)—$ in which $R^7$ represents an alkyl group; or $—(CNR^8)—$ in which $R^8$ represents a group $OR^4$ or $HNR^4$; and the other of A and B represents a group $—CR^9R^{11}—CR^{10}R^{12}—$, in which each of $R^9$, $R^{10}$, $R^{11}$ and $R^{12}$ independently represents a hydrogen atom or an alkyl group, or $R^{11}$ and $R^{12}$ together represent a carbon- carbon bond, a $—CH_2—$ group or an oxygen atom; but, in a method of combating undesired plant growth, is not a compound of formula I wherein $R^1$, X and Y are all hydrogen atoms, Ar is unsubstituted phenyl and the linkage —A—B— is $—CO—CH(CH_3)—CH_2—$ or $—(CH_2)_2—CO—$.

**Claims for the Contracting States: LU SE**

1. A compound of the general formula

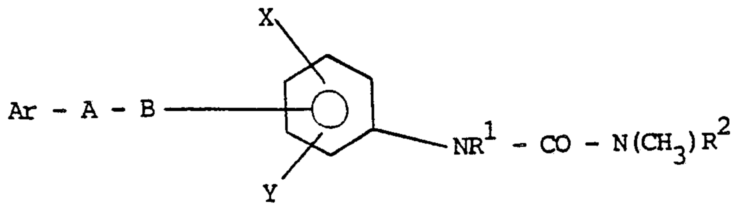

(I)

in which $R^1$ represents a hydrogen atom or an alkyl group having 1 to 4 carbon atoms; $R^2$ represents a hydrogen atom, a methoxy or a methyl group; Ar represents an optionally substituted phenyl group or an optionally substituted heteroaromatic ring containing 1 or 2 oxygen, sulphur and/or nitrogen atoms, optional substituents being selected from halogen atoms and alkyl, alkoxy, haloalkyl, haloalkoxy, alkylthio, phenyl, phenoxy, cyano, amino, alkylamino, amido, hydroxy, carboxy and alkoxycarbonyl groups, alkyl moieties in such substituents having up to 4 carbon atoms; each of X and Y independently represents a hydrogen or halogen atom or an alkyl, haloalkyl or alkoxy group; and one of A and B represents a group —CO—; —$CR^3(OR^4)$— or —$CR^3(SR^4)$—, in which $R^3$ represents a hydrogen atom or an alkyl group and $R^4$ represents a hydrogen atom, an alkyl, alkenyl, alkynyl or cycloalkyl group, an optionally substituted phenyl or benzyl group, optional substituents being selected from halogen atoms and alkyl, alkoxy, haloalkyl, haloalkoxy, alkylthio, phenyl, phenoxy, cyano, amino, alkylamino, amido, hydroxy, carboxy and alkoxycarbonyl groups, alkyl moieties in such substituents having up to 4 carbon atoms; or an acyl group derived from a carboxylic, carbamic, sulphonic or phosphoric acid; $C(OR^5)(OR^6)$—, —$C(OR^5)(SR^6)$— or —$C(SR^5)(SR^6)$—, in which each of $R^5$ and $R^6$ independently represents an alkyl group or together represent an alkylene group; —$CH(CH_2COOR^7)$— in which $R^7$ represents an alkyl group; or —$(CNR^8)$— in which $R^8$ represents a group $OR^4$ or $HNR^4$; and the other of A and B represents a group —$CR^9R^{11}$—$CR^{10}R^{12}$—, in which each of $R^9$, $R^{10}$, $R^{11}$ and $R^{12}$ independently represents a hydrogen atom or an alkyl group, or $R^{11}$ and $R^{12}$ together represent a carbon- carbon bond, a —$CH_2$— group or an oxygen atom.

2. A compound as claimed in claim 1, in which $R^1$ represents a methyl group or a hydrogen atom.

3. A compound as claimed in claims 1 or 2, in which Ar represents an unsubstituted pyridine ring or a phenyl group which is unsubstituted or substituted by one or more substituents selected from halogen atoms and $C_{1-4}$ alkyl, alkoxy and haloalkyl groups.

4. A compound as claimed in any one of claims 1 to 3, in which both X and Y represent hydrogen atoms.

5. A compound as claimed in any one of claims 1 to 4, in which one of A and B represents a group —CO—, —$CR^3(OR^4)$—, —$C(OR^5)(OR^6)$— or —$C(SR^5)(SR^6)$—.

6. A compound as claimed in any preceding claim, in which $R^3$ represents a hydrogen atom.

7. A compound as claimed in any preceding claim wherein $R^4$ represents a hydrogen atom or a $C_{1-4}$ alkyl, ($C_{1-4}$ alkyl)— carbonyl or methylsulphonyl group; and either each of $R^5$ and $R^6$ independently represents a $C_{1-4}$ alkyl group or $R^5$ and $R^6$ together represent a dimethylene or trimethylene group.

8. A compound as claimed in any preceding claim, in which one of A and B represents a group —CO—, —CH(OH)—, —$CH(OCH_3)$ or

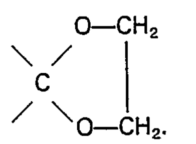

9. A compound as claimed in any one of claims 1 to 8, in which one of A and B represents a group —CH=CH— or —$CH_2$—$CH_2$—.

10. A compound as claimed in claim 5, 6 or 7 and in claim 8, wherein A represents the group of formula —CO—, —$CR^3(OR^4)$—, —$C(OR^5)(OR^6)$ or —$C(SR^5)(SR^6)$ and B represents the group of formula —CH=CH— or —$CH_2$—$CH_2$.

11. A compound according to any preceding claim, wherein $R^2$ represents a methyl group.

12. A process for the preparation of a compound as claimed in claim 1, which comprises reacting a compound of the general formula:

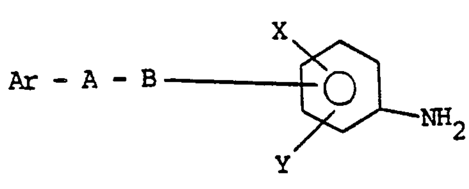

(II)

in which Ar, A, B, X and Y have the meanings given for the general formula I, with either

(a) a compound of the general formula:

$$Hal—CO—N(CH_3)R^2 \qquad (III)$$

in which $R^2$ has the meaning given for the general formula I and Hal represents a halogen atom; or

(b) phosgene, to produce the corresponding isocyanate, and reacting at least part of the isocyanate with an amine of the general formula:

$$HN(CH_3)R^2 \qquad (IV)$$

in which formula $R^2$ has the meaning given for the general formula I; to produce a compound of the general formula I in which $R^1$ represents a hydrogen atom; and if desired, converting the resulting compound of the general formula I into any other desired compound of the general formula I.

13. A herbicidal and/or fungicidal composition which comprises a compound as claimed in any one of claims 1 to 10 together with a carrier.

14. A compound as claimed in claim 13, which comprises at least two carriers, at least one of which is a surface-active agent.

15. A method of combating fungi and/or undesired plant growth at a locus which comprises applying to the locus a compound as claimed in any one of claims 1 to 10 or a composition as claimed in either claim 13 or 14.

**Patentansprüche für die Vertragsstaaten: BE CH DE FR GB IT LI NL**

1. Verbindung der allgemeinen Formel

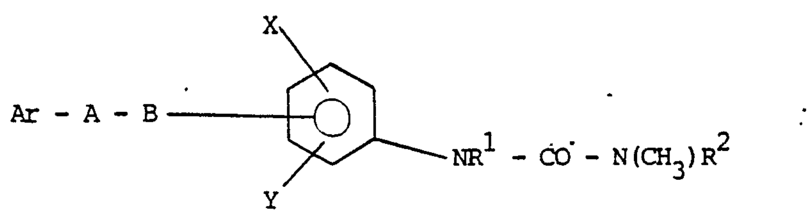

(I)

worin $R^1$ ein Wasserstoffatom oder eine Alkylgruppe mit 1 bis 4 Kohlenstoffatomen bedeutet; $R^2$ eine Methylgruppe darstellt; Ar eine gegebenenfalls substituierte Phenylgruppe oder einen gegebenenfalls substituierten heteroaromatischen Ring mit 1 oder 2 Sauerstoff-, Schwefel- und/oder Stickstoffatomen bedeutet, worin fakultative Substituenten ausgewählt sind unter Halogenatomen und Alkyl-, Alkoxy-, Halogenalkyl-, Halogenalkoxy-, Alkylthio-, Phenyl-, Phenoxy-, Cyano-, Amino-, Alkylamin-, Amido-, Hydroxy-, Carboxy- und Alkoxycarbonylgruppen, wobei Alkylreste in solchen Substituenten bis zu 4 Kohlenstoffatomen aufweisen; jeder der Reste X und Y unabhängig voneinander ein Wasserstoff- oder Halogenatom oder eine Alkyl-, Halogenalkyl- oder Alkoxygruppe darstellt; und einer der Reste A und B eine Gruppe —CO—; $—CR^3(OR^4)—$ oder $—CR^3(SR^4)—$ bedeutet, worin $R^3$ ein Wasserstoffatom oder eine Alkylgruppe darstellt und $R^4$ ein Wasserstoffatom, eine Alkyl-, Alkenyl-, Alkinyl- oder Cycloalkylgruppe oder eine gegebenenfalls substituierte Phenyl- oder Benzylgruppe darstellt, worin fakultative Substituenten ausgewählt sind unter Halogenatom und Alkyl-, Alkoxy-, Halogenalkyl-, Alkylthio-, Phenyl-, Phenoxy-, Cyano-, Amino-, Alkylamino-, Amido-, Hydroxy-, Carboxy- und Alkoxycarbonylgruppen, wobei Alkylreste in solchen Substituenten bis zu 4 Kohlenstoffatome aufweisen; oder eine von einer Carbonsäure, Carbaminsäure, Sulfonsäure oder Phosphorsäure abgeleitete Acylgruppe darstellt; $—C(OR^5)(OR^6)—$, $—C(OR^5)(SR^6)—$ oder $—C(SR^5)(SR^6)—$ darstellt, worin jeder Rest $R^5$ und $R^6$ unabhängig voneinander eine Alkylgruppe bedeutet oder die Reste zusammen eine Alkylengruppe darstellen; $—CH(CH_2COOR^7)—$ darstellt, worin $R^7$ eine Alkylgruppe bedeutet; oder $—CNR^8)—$ darstellt, worin $R^8$ eine Gruppe $OR^4$ oder $NHR^4$ bedeutet; und der andere der Reste A und B eine Gruppe $—CR^9R^{11}—CR^{10}R^{12}—$ darstellt, worin jeder der Reste $R^9$, $R^{10}$, $R^{11}$ und $R^{12}$ unabhängig voneinander ein Wasserstoffatom oder eine Alkylgruppe bedeutet oder $R^{11}$ und $R^{12}$ zusammen eine Kohlenstoff-Kohlenstoff-Bindung, eine $—CH_2$-Gruppe oder ein Sauerstoffatom darstellen; jedoch unter Ausschluß von Verbindungen der Formel I, worin $R^1$, X und Y sämtlich Wasserstoffatome bedeuten, Ar unsubstituiertes Phenyl ist und die Verknüpfung —A—B— für $—CO—CH(CH_3)—CH_2—$ oder $—(CH_2)_2—CO—$ steht.

2. Verbindung nach Anspruch 1, worin $R^1$ eine Methylgruppe oder ein Wasserstoffatom bedeutet.

3. Verbindung nach Anspruch 1 oder 2, worin Ar einen unsubstituierten Pyridinring oder eine Phenylgruppe bedeutet, die unsubstituiert ist oder durch einen oder mehrere Substituenten, ausgewählt unter Halogenatom und $C_{1-4}$Alkyl-, Alkoxy- und Halogenalkylgruppen, substituiert ist.

4. Verbindung nach einem der Ansprüche 1 bis 3, worin sowohl X als auch Y Wasserstoffatome bedeuten.

5. Verbindung nach einem der Ansprüche 1 bis 4, worin einer der Reste A und B eine Gruppe —CO—, —$CR^3(OR^4)$—, —$C(OR^5)(OR^6)$— oder —$C(SR^5)$ $(SR^6)$— bedeutet.

6. Verbindung nach einem vorstehenden Anspruch, worin $R^3$ ein Wasserstoffatom bedeutet.

7. Verbindung nach einem vorstehenden Anspruch, worin $R^4$ ein Wasserstoffatom oder eine $C_{1-4}$Alkylgruppe, ($C_{1-4}$Alkyl)carbonylgruppe oder eine Methylsulfonylgruppe bedeutet; und entweder jeder der Reste $R^5$ und $R^6$ unabhängig voneinander eine $C_{1-4}$Alkylgruppe darstellt oder $R^5$ und $R^6$ gemeinsam eine Dimethylen- oder Trimethylengruppe bedeuten.

8. Verbindung nach einem vorstehenden Anspruch, worin einer der Reste A und B eine Gruppe —CO—, —CH(OH)—, —$CH(OCH_3)$ oder

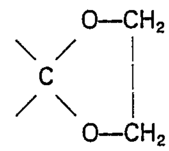

darstellt.

9. Verbindung nach einem der Ansprüche 1 bis 8, worin einer der Reste A und B eine Gruppe —CH=CH— oder $CH_2$—$CH_2$— darstellt.

10. Verbindung nach Anspruch 5, 6, 7 oder 8, worin A eine Gruppe der Formel—CO—, —$CR^3(OR^4)$—, —$C(OR^5)$ $(OR^6)$ oder —$C(SR^5)$ $(SR^6)$ und B eine Gruppe der Formel —CH=CH— oder —$CH_2$—$CH_2$— bedeutet.

11. Verfahren zur Herstellung einer Verbindung nach Anspruch 1, welches ein Umsetzen einer Verbindung der allgemeinen Formel:

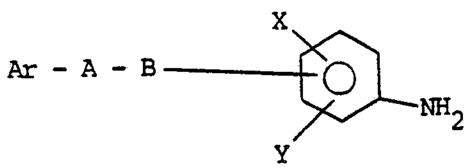

(II)

worin Ar, A, B, X und Y die für die allgemeine Formel I angegebenen Bedeutungen besitzen, entweder mit

(a) einer Verbindung der allgemeinen Formel:

$$Hal — CO — N(CH_3)R^2 \quad , \qquad (III)$$

worin $R^2$ die für die allgemeine Formel I angegebene Bedeutung aufweist und Hal ein Halogenatom, bedeutet; oder

(b) mit Phosgen ($COCl_2$) zur Ausbildung des entsprechenden Isocyanats und ein Umsetzen wenigstens eines Teiles des Isocyanats mit einem Amin der allgemeinen Formel:

$$HN(CH_3)R^2 \quad , \qquad (IV)$$

worin $R^2$ die für die allgemeine Formel I angegebene Bedeutung hat; zur Ausbildung einer Verbindung der allgemeinen Formel I, worin $R^1$ ein Wasserstoffatomen bedeutet; und gewünschtenfalls ein Überführen der erhaltenen Verbindung der allgemeinen Formel I in eine andere gewünschte Verbindung der allgemeinen Formel I umfaßt.

12. Herbizide und/oder fungizide Zusammensetzung, welche eine Verbindung, wie in einem der Ansprüche 1 bis 10 beansprucht, zusammen mit einem Träger umfaßt.

13. Zusammensetzung nach Anspruch 12, welche wenigstens zwei Träger umfaßt, von denen wenigstens einer ein oberflächenaktives Mittel ist.

14. Verfahren zur Bekämpfung von Pilzen und/oder von unerwünschtem Pflanzenwuchs an einem Ort, welches ein Auftragen einer Verbindung der allgemeinen Formel

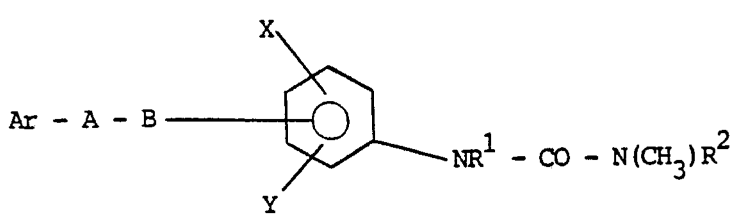

(I)

auf den Ort umfaßt, in welcher Formel $R^1$ ein Wasserstoffatom oder eine Alkylgruppe mit 1 bis 4 Kohlenstoffatomen bedeutet; $R^2$ eine Methylgruppe darstellt; Ar eine gegebenenfalls substituierte

Phenylgruppe oder einen gegebenenfalls substituierten heteroaromatischen Ring mit 1 oder 2 Sauerstoff-, Schwefel- und/oder Stickstoffatomen bedeutet, worin fakultative Substituenten ausgewählt sind unter Halogenatomen und Alkyl-, Alkoxy-, Halogenalkyl-, Halogenalkoxy-, Alkylthio-, Phenyl-, Phenoxy-, Cyano-, Amino-, Alkylamin-, Amido-, Hydroxy-, Carboxy- und Alkoxycarbonylgruppen, wobei Alkylreste in solchen Substituenten bis zu 4 Kohlenstoffatomen aufweisen; jeder der Reste X und Y unabhängig voneinander ein Wasserstoff- oder Halogenatom oder eine Alkyl-, Halogenalkyl- oder Alkoxygruppe darstellt; und einer der Reste A und B eine Gruppe —CO—; —$CR^3(OR^4)$— oder —$CR^3(SR^4)$— bedeutet, worin $R^3$ ein Wasserstoffatom oder eine Alkylgruppe darstellt und $R^4$ ein Wasserstoffatom, eine Alkyl-, Alkenyl-, Alkinyl- oder Cycloalkylgruppe oder eine gegebenenfalls substituierte Phenyl- oder Benzylgruppe darstellt, worin fakultative Substituenten ausgewählt sind unter Halogenatom und Alkyl-, Alkoxy-, Halogenalkyl-, Alkylthio-, Phenyl-, Phenoxy-, Cyano-, Amino-, Alkylamino-, Amido-, Hydroxy-, Carboxy- und Alkoxycarbonylgruppen, wobei Alkylreste in solchen Substituenten bis zu 4 Kohlenstoffatome aufweisen; oder eine von einer Carbonsäure, Carbaminsäure, Sulfonsäure oder Phosphorsäure abgeleitete Acylgruppe darstellt; —$C(OR^5)(OR^6)$—, —$C(OR^5)(SR^6)$— oder —$C(SR^5)(SR^6)$— darstellt, worin jeder Rest $R^5$ und $R^6$ unabhängig voneinander eine Alkylgruppe bedeutet oder die Reste zusammen eine Alkylengruppe darstellen; —$CH(CH_2COOR^7)$— darstellt, worin $R^7$ eine Alkylgruppe bedeutet; oder —$CNR^8$)— darstellt, worin $R^8$ eine Gruppe $OR^4$ oder $NHR^4$ bedeutet; und der andere der Reste A und B eine Gruppe —$CR^9R^{11}$—$CR^{10}R^{12}$— darstellt, worin jeder der Reste $R^9$, $R^{10}$, $R^{11}$ und $R^{12}$ unabhängig voneinander ein Wasserstoffatom oder eine Alkylgruppe bedeutet oder $R^{11}$ und $R^{12}$ zusammen eine Kohlenstoff-Kohlenstoff-Bindung, eine —$CH_2$-Gruppe oder ein Sauerstoffatom darstellen; jedoch, in einem Verfahren zur Bekämpfung von unerwünschten Pflanzenwuchs, nicht eine Verbindung der Formel I ist, worin $R^1$, X und Y sämtlich Wasserstoffatome bedeuten, Ar unsubstituiertes Phenyl ist und die Verknüpfung —A—B— für —CO—$CH(CH_3)$—$CH_2$— oder —$(CH_2)_2$—CO— steht.

**Patentansprüche für den Vertragsstaat: AT**

1. Herbizide und/oder fungizide Zusammensetzung, welche einen wirksamen Bestandteil zusammen mit einem Träger umfaßt, dadurch gekennzeichnet, daß der wirksame Bestandteil eine Verbindung der allgemeinen Formel

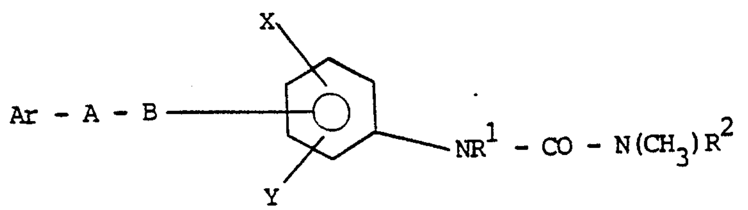

(I)

ist, worin $R^1$ ein Wasserstoffatom oder eine Alkylgruppe mit 1 bis 4 Kohlenstoffatomen bedeutet; $R^2$ eine Methylgruppe darstellt; Ar eine gegebenenfalls substituierte Phenylgruppe oder einen gegebenenfalls substituierten heteroaromatischen Ring mit 1 oder 2 Sauerstoff-, Schwefel- und/oder Stickstoffatomen bedeutet, worin fakultative Substituenten ausgewählt sind unter Halogenatomen und Alkyl-, Alkoxy-, Halogenalkyl-, Halogenalkoxy-, Alkylthio-, Phenyl-, Phenoxy-, Cyano-, Amino-, Alkylamin-, Amido-, Hydroxy-, Carboxy- und Alkoxycarbonylgruppen, wobei Alkylreste in solchen Substituenten bis zu 4 Kohlenstoffatomen aufweisen; jeder der Reste X und Y unabhängig voneinander ein Wasserstoff- oder Halogenatom oder eine Alkyl-, Halogenalkyl- oder Alkoxygruppe darstellt; und einer der Reste A und B eine Gruppe —CO—; —$CR^3(OR^4)$— oder —$CR^3(SR^4)$— bedeutet, worin $R^3$ ein Wasserstoffatom oder eine Alkylgruppe darstellt und $R^4$ ein Wasserstoffatom, eine Alkyl-, Alkenyl-, Alkinyl- oder Cycloalkylgruppe oder eine gegebenenfalls substituierte Phenyl- oder Benzylgruppe darstellt, worin fakultative Substituenten ausgewählt sind unter Halogenatomen und Alkyl-, Alkoxy-, Halogenalkyl-, Alkylthio-, Phenyl-, Phenoxy-, Cyano-, Amino-, Alkylamino-, Amido-, Hydroxy-, Carboxy- und Alkoxycarbonylgruppen, wobei Alkylreste in solchen Substituenten bis zu 4 Kohlenstoffatome aufweisen; oder eine von einer Carbonsäure, Carbaminsäure, Sulfonsäure oder Phosphorsäure abgeleitete Acylgruppe darstellt; —$C(OR^5)(OR^6)$—, —$C(OR^5)(SR^6)$— oder —$C(SR^5)(SR^6)$— darstellt, worin jeder Rest $R^5$ und $R^6$ unabhängig voneinander eine Alkylgruppe bedeutet oder die Reste zusammen eine Alkylengruppe darstellen; —$CH(CH_2COOR^7)$— darstellt, worin $R^7$ eine Alkylgruppe bedeutet; oder —$(CNR^8)$— darstellt, worin $R^8$ eine Gruppe $OR^4$ oder $NHR^4$ bedeutet; und der andere der Reste A und B eine Gruppe —$CR^9R^{11}$—$CR^{10}R^{12}$— darstellt, worin jeder der Reste $R^9$, $R^{10}$, $R^{11}$ und $R^{12}$ unabhängig voneinander ein Wasserstoffatom oder eine Alkylgruppe bedeutet oder $R^{11}$ und $R^{12}$ zusammen eine Kohlenstoff-Kohlenstoff-Bindung, eine —$CH_2$-Gruppe oder ein Sauerstoffatom darstellen; jedoch unter Ausschluß von Verbindungen der Formel I, worin $R^1$, X und Y sämtlich Wasserstoffatome bedeuten, Ar unsubstituiertes Phenyl ist und die Verknüpfung —A—B— für —CO—$CH(CH_3)$—$CH_2$— oder —$(CH_2)_2$—CO— steht.

2. Zusammensetzung nach Anspruch 1, worin $R^1$ eine Methylgruppe oder ein Wasserstoffatom bedeutet.

3. Zusammensetzung nach Anspruch 1 oder 2, worin Ar einen unsubstituierten Pyridinring oder eine

Phenylgruppe bedeutet, die unsubstituiert ist oder durch einen oder mehrere Substituenten, ausgewählt unter Halogenatom und $C_{1-4}$Alkyl-, Alkoxy- und Halogenalkylgruppen, substituiert ist.

4. Zusammensetzung nach einem der Ansprüche 1 bis 3, worin sowohl X als auch Y Wasserstoffatome bedeuten.

5. Zusammensetzung nach einem der Ansprüche 1 bis 4, worin einer der Reste A und B eine Gruppe —CO—, $—CR^3(OR^4)—$, $—C(OR^5)\ (OR^6)—$ oder $—C(SR^5)\ (SR^6)—$ bedeutet.

6. Zusammensetzung nach einem vorstehenden Anspruch, worin $R^3$ ein Wasserstoffatom bedeutet.

7. Zusammensetzung nach einem vorstehenden Anspruch, worin $R^4$ ein Wasserstoffatom oder eine $C_{1-4}$Alkylgruppe, ($C_{1-4}$Alkyl)carbonylgruppe oder eine Methylsulfonylgruppe bedeutet; und entweder jeder der Reste $R^5$ und $R^6$ unabhängig voneinander eine $C_{1-4}$Alkylgruppe darstellt oder $R^5$ und $R^6$ gemeinsam eine Dimethylen- oder Trimethylengruppe bedeuten.

8. Zusammensetzung nach einem vorstehenden Anspruch, worin einer der Reste A und B eine Gruppe —CO—, —CH(OH)—, $—CH(OCH_3)$ oder

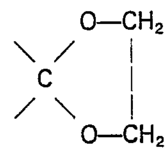

darstellt.

9. Zusammensetzung nach einem der Ansprüche 1 bis 8, worin einer der Reste A und B eine Gruppe —CH=CH— oder $CH_2—CH_2—$ darstellt.

10. Zusammensetzung nach Anspruch 5, 6, 7 oder 8, worin A eine Gruppe der Formel —CO—, $—CR^3(OR^4)—$, $—C(OR^5)\ (OR^6)$ oder $—C(SR^5)\ (SR^6)$ und B eine Gruppe der Formel —CH=CH— oder $—CH_2—CH_2—$ bedeutet.

11. Zusammensetzung nach einem vorstehenden Anspruch, welche wenigstens zwei Träger umfaßt, von denen wenigstens einer ein oberflächenaktives Mittel ist.

12. Verfahren zur Herstellung einer Verbindung der Formel I, wie in Anspruch 1 definiert, welches ein Umsetzen einer Verbindung der allgemeinen Formel:

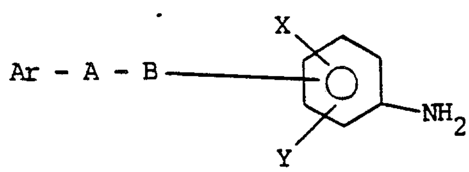

(II)

worin Ar, A, B, X und Y die für die allgemeine Formel I angegebenen Bedeutungen besitzen, entweder mit

(a) einer Verbindung der allgemeinen Formel:

$$Hal—CO—N(CH_3)R^2 \quad , \qquad (III)$$

worin $R^2$ die für die allgemeine Formel I angegebene Bedeutung aufweist und Hal ein Halogenatom, bedeutet; oder

(b) mit Phosgen ($COCl_2$) zur Ausbildung des entsprechenden Isocyanats und ein Umsetzen wenigstens eines Teiles des Isocyanats mit einem Amin der allgemeinen Formel:

$$HN(CH_3)R^2 \quad , \qquad (IV)$$

worin $R^2$ die für die allgemeine Formel I angegebene Bedeutung hat;

zur Ausbildung einer Verbindung der allgemeinen Formel I, worin $R^1$ ein Wasserstoffatom bedeutet; und gewünschtenfalls ein Überführen der erhaltenen Verbindung der allgemeinen Formel I in eine andere gewünschte Verbindung der allgemeinen Formel I umfaßt.

13. Verfahren zur Bekämpfung von Pilzen und/oder von unerwünschtem, Pflanzenwuchs an einem Ort, welches ein Aufbringen einer Zusammensetzung, worin der wirksame Bestandteil eine Verbindung der allgemeinen Formel

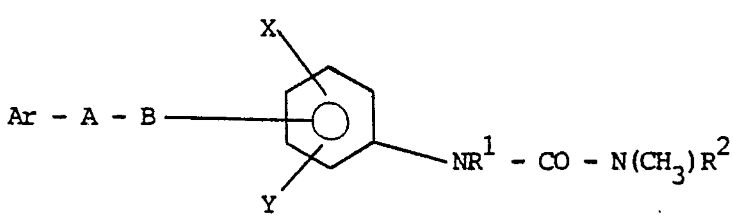

(I)

ist, auf den Ort umfaßt, in welcher Formel $R^1$ ein Wasserstoffatom oder eine Alkylgruppe mit 1 bis 4 Kohlenstoffatomen bedeutet; $R^2$ eine Methylgruppe darstellt; Ar eine gegebenenfalls substituierte Phenylgruppe oder einen gegebenenfalls substituierten heteroaromatischen Ring mit 1 oder 2 Sauerstoff-, Schwefel- und/oder Stickstoffatomen bedeutet, worin fakultative Substituenten ausgewählt sind unter Halogenatomen und Alkyl-, Alkoxy-, Halogenalkyl-, Halogenalkoxy-, Alkylthio-, Phenyl-, Phenoxy-, Cyano-, Amino-, Alkylamino-, Amido-, Hydroxy-, Carboxy- und Alkoxycarbonylgruppen, wobei Alkylreste in solchen Substituenten bis zu 4 Kohlenstoffatomen aufweisen; jeder der Reste X und Y unabhängig voneinander ein Wasserstoff- oder Halogenatom oder eine Alkyl-, Halogenalkyl- oder Alkoxygruppe darstellt; und einer der Reste A und B eine Gruppe —CO—; —$CR^3(OR^4)$— oder —$CR^3(SR^4)$— bedeutet, worin $R^3$ ein Wasserstoffatom oder eine Alkylgruppe darstellt und $R^4$ ein Wasserstoffatom, eine Alkyl-, Alkenyl-, Alkinyl- oder Cycloalkylgruppe oder eine gegebenenfalls substituierte Phenyl- oder Benzylgruppe darstellt, worin fakultative Substituenten ausgewählt sind unter Halogenatomen und Alkyl-, Alkoxy-, Halogenalkyl-, Halogenalkoxy-, Alkylthio-, Phenyl-, Phenoxy-, Cyano-, Amino-, Alkylamino-, Amido-, Hydroxy-, Carboxy- und Alkoxycarbonylgruppen, wobei Alkylreste in solchen Substituenten bis zu 4 Kohlenstoffatome aufweisen; oder eine von einer Carbonsäure, Carbaminsäure, Sulfonsäure oder Phosphorsäure abgeleitete Acylgruppe darstellt; —$C(OR^5)(OR^6)$—, —$C(OR^5)(SR^6)$— oder —$C(SR^5)(SR^6)$— darstellt, worin jeder Rest $R^5$ und $R^6$ unabhängig voneinander eine Alkylgruppe bedeutet oder die Reste zusammen eine Alkylengruppe darstellen; —$CH(CH_2COOR^7)$— darstellt, worin $R^7$ eine Alkylgruppe bedeutet; oder —$(CNR^8)$— darstellt, worin $R^8$ eine Gruppe $OR^4$ oder $NHR^4$ bedeutet; und der andere der Reste A und B eine Gruppe —$CR^9R^{11}$—$CR^{10}R^{12}$— darstellt, worin jeder der Reste $R^9$, $R^{10}$, $R^{11}$ und $R^{12}$ unabhängig voneinander ein Wasserstoffatom oder eine Alkylgruppe bedeutet oder $R^{11}$ und $R^{12}$ zusammen eine Kohlenstoff-Kohlenstoff-Bindung, eine —$CH_2$-Gruppe oder ein Sauerstoffatom darstellen; jedoch, in einem Verfahren zur Bekämpfung von unerwünschtem Pflanzenwuchs, nicht eine Verbindung der Formel I ist, worin $R^1$, X und Y sämtlich Wasserstoffatome bedeuten, Ar unsubstituiertes Phenyl ist und die Verknüpfung —A—B— für —CO—$CH(CH_3)$—$CH_2$— oder —$(CH_2)_2$—CO— steht.

**Patentansprüche für die Vertragsstaaten: LU SE**

1. Verbindung der allgemeinen Formel

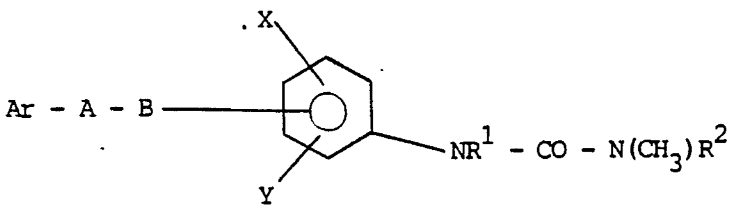

(I)

worin $R^1$ ein Wasserstoffatom oder eine Alkylgruppe mit 1 bis 4 Kohlenstoffatomen bedeutet; $R^2$ ein Wasserstoffatom eine methoxy- oder Methylgruppe darstellt; Ar eine gegebenenfalls substituierte Phenylgruppe oder einen gegebenenfalls substituierten heteroaromatischen Ring mit 1 oder 2 Sauerstoff-, Schwefel- und/oder Stickstoffatomen bedeutet, worin fakultative Substituenten ausgewählt sind unter Halogenatomen und Alkyl-, Alkoxy-, Halogenalkyl-, Halogenalkoxy-, Alkylthio-, Phenyl-, Phenoxy-, Cyano-, Amino-, Alkylamin-, Amido-, Hydroxy-, Carboxy- und Alkoxycarbonylgruppen, wobei Alkylreste in solchen Substituenten bis zu 4 Kohlenstoffatomen aufweisen; jeder der Reste X und Y unabhängig voneinander ein Wasserstoff- oder Halogenatom oder eine Alkyl-, Halogenalkyl- oder Alkoxygruppe darstellt; und einer der Reste A und B eine Gruppe —CO—; —$CR^3(OR^4)$— oder —$CR^3(SR^4)$— bedeutet, worin $R^3$ ein Wasserstoffatom oder eine Alkylgruppe darstellt und $R^4$ ein Wasserstoffatom, eine Alkyl-, Alkenyl-, Alkinyl- oder Cycloalkylgruppe oder eine gegebenenfalls substituierte Phenyl- oder Benzylgruppe darstellt, worin fakultative Substituenten ausgewählt sind unter Halogenatom und Alkyl-, Alkoxy-, Halogenalkyl-, Alkylthio-, Phenyl-, Phenoxy-, Cyano-, Amino-, Alkylamino-, Amido-, Hydroxy-, Carboxy- und Alkoxycarbonylgruppen, wobei Alkylreste in solchen Substituenten bis zu 4 Kohlenstoffatome aufweisen; oder eine von einer Carbonsäure, Carbaminsäure, Sulfonsäure oder Phosphorsäure abgeleitete Acylgruppe darstellt; —$C(OR^5)(OR^6)$—, —$C(OR^5)(SR^6)$— oder —$C(SR^5)(SR^6)$— darstellt, worin jeder Rest $R^5$ und $R^6$ unabhängig voneinander eine Alkylgruppe bedeutet oder die Reste zusammen eine Alkylengruppe darstellen; —$CH(CH_2COOR^7)$— darstellt, worin $R^7$ eine Alkylgruppe bedeutet; oder —$(CNR^8)$— darstellt, worin $R^8$ eine Gruppe $OR^4$ oder $NHR^4$ bedeutet; und der andere der Reste A und B eine Gruppe —$CR^9R^{11}$—$CR^{10}R^{12}$— darstellt, worin jeder der Reste $R^9$, $R^{10}$, $R^{11}$ und $R^{12}$ unabhängig voneinander ein Wasserstoffatom oder eine Alkylgruppe bedeutet oder $R^{11}$ und $R^{12}$ zusammen eine Kohlenstoff-Kohlenstoff-Bindung, eine —$CH_2$-Gruppe oder ein Sauerstoffatom darstellen.

2. Verbindung nach Anspruch 1, worin $R^1$ eine Methylgruppe oder ein Wasserstoffatom bedeutet.

3. Verbindung nach Anspruch 1 oder 2, worin Ar einen unsubstituierten Pyridinring oder eine Phenylgruppe bedeutet, die unsubstituiert ist oder durch einen oder mehrere Substituenten, ausgewählt unter Halogenatom und $C_{1-4}$Alkyl-, Alkoxy- und Halogenalkylgruppen, substituiert ist.

4. Verbindung nach einem der Ansprüche 1 bis 3, worin sowohl X als auch Y Wasserstoffatome bedeuten.

5. Verbindung nach einem der Ansprüche 1 bis 4, worin einer der Reste A und B eine Gruppe —CO—, $—CR^3(OR^4)—$, $—C(OR^5)(OR^6)—$ oder $—C(SR^5)(SR^6)—$ bedeutet.

6. Verbindung nach einem vorstehenden Anspruch, worin $R^3$ ein Wasserstoffatom bedeutet.

7. Verbindung nach einem vorstehenden Anspruch, worin $R^4$ ein Wasserstoffatom oder eine $C_{1-4}$Alkylgruppe, ($C_{1-4}$Alkyl)carbonylgruppe oder eine Methylsulfonylgruppe bedeutet; und entweder jeder der Reste $R^5$ und $R^6$ unabhängig voneinander eine $C_{1-4}$Alkylgruppe darstellt oder $R^5$ und $R^6$ gemeinsam eine Dimethylen- oder Trimethylengruppe bedeuten.

8. Verbindung nach einem vorstehenden Anspruch, worin einer der Reste A und B eine Gruppe —CO—, —CH(OH)—, $—CH(OCH_3)$ oder

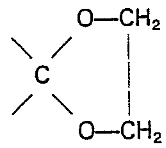

darstellt.

9. Verbindung nach einem der Ansprüche 1 bis 8, worin einer der Reste A und B eine Gruppe —CH=CH— oder $CH_2—CH_2—$ darstellt.

10. Verbindung nach Anspruch 5, 6, 7 oder 8, worin A eine Gruppe der Formel—CO—, $—CR^3(OR^4)—$, $—C(OR^5)(OR^6)$ oder $—C(SR^5)(SR^6)$ und B eine Gruppe der Formel —CH=CH— oder $—CH_2—CH_2—$ bedeutet.

11. Verbindung nach einem vorstehenden Anspruch, worin $R^2$ eine Methylgruppe bedeutet.

12. Verfahren zur Herstellung einer Verbindung nach Anspruch 1, welches ein Umsetzen einer Verbindung der allgemeinen Formel:

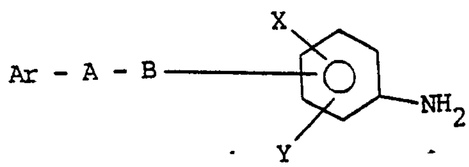

(II)

worin Ar, A, B, X und Y die für die allgemeine Formel I angegebenen Bedeutungen besitzen, entweder mit

(a) einer Verbindung der allgemeinen Formel:

$$Hal — CO — N(CH_3)R^2 \quad , \qquad (III)$$

worin $R^2$ die für die allgemeine Formel I angegebene Bedeutung aufweist und Hal ein Halogenatom, bedeutet; oder

(b) mit Phosgen ($COCl_2$) zur Ausbildung des entsprechenden Isocyanats und ein Umsetzen wenigstens eines Teiles des Isocyanats mit einem Amin der allgemeinen Formel:

$$HN(CH_3)R^2 \quad , \qquad (IV)$$

worin $R^2$ die für die allgemeine Formel I angegebene Bedeutung hat;
zur Ausbildung einer Verbindung der allgemeinen Formel I, worin $R^1$ ein Wasserstoffatom bedeutet; und gewünschtenfalls ein Überführen der erhaltenen Verbindung der allgemeinen Formel I in eine andere gewünschte Verbindung der allgemeinen Formel I umfaßt.

13. Herbizide und/oder fungizide Zusammensetzung, welche eine Verbindung, wie in einem der Ansprüche 1 bis 10 beansprucht, zusammen mit einem Träger umfaßt.

14. Zusammensetzung nach Anspruch 13, welche wenigstens zwei Träger umfaßt, von denen wenigstens einer ein oberflächenaktives Mittel ist.

15. Verfahren zur Bekämpfung von Pilzen und/oder von unerwünschtem Pflanzenwuchs an einem Ort, welches ein Auftragen einer Verbindung, wie in einem der Ansprüche 1 bis 10 beansprucht, oder einer Zusammensetzung, wie in Anspruch 13 oder 14 beansprucht, auf den Ort umfaßt.

**Revendications pour les Etats contractants: BE CH DE FR GB IT NL**

1. Un composé de la formule générale

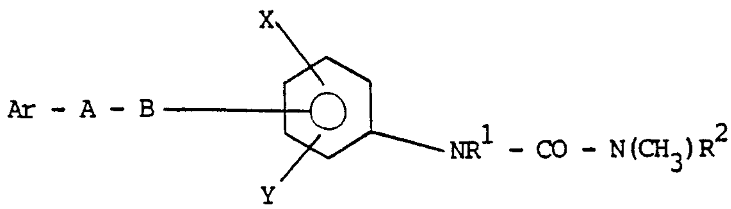

(I)

dans laquelle $R^1$ représente un atome d'hydrogène ou un groupe alcoyle ayant 1 à 4 atomes de carbone; $R^2$ représente un groupe méthyle; Ar représente un groupe phényle éventuellement substitué ou un noyau hétéro aromatique éventuellement substitué contenant 1 ou 2 atomes d'oxygène, de soufre et/ou d'azote, les substituants éventuels étant choisis parmi des atomes d'halogènes et des groupes alcoyle, alcoxy, haloalcoyle, haloalcoxy, alcoylthio, phényle, phénoxy, cyano, amino, alcoylamino, amido, hydroxy, carboxy et alcoxycarbonyle, les portions alcoyle dans ces substituants ayant jusqu'à 4 atomes de carbone; chacun de X et Y indépendamment représente un atome d'hydrogène ou d'halogène ou un groupe alcoyle, haloalcoyle ou alcoxy; et un de A et B représente un groupe $—CO—$; $—CR^3(OR^4)—$ ou $—CR^3(SR^4)—$, où $R^3$ représente un atome d'hydrogène ou un groupe alcoyle et $R^4$ représente un atome d'hydrogène ou un d'hydrogène, ou un groupe alcoyle, alcényle, alcynyle ou cycloalcoyle, un groupe phényle ou benzyle éventuellement substitué, les substituants éventuels étant choisis parmi des atomes d'halogènes et des groupes alcoyle, alcoxy, haloalcoyle, haloalcoxy, alcoylthio, phényle, phénoxy, cyano, amino, alcoylamino, amido, hydroxy, carboxy et alcoxycarbonyle, les portions alcoyle dans ces substituants ayant jusqu'à 4 atomes de carbone; ou un groupe acyle dérivé d'un acide carboxylique, carbamique, sulfonique ou phosphorique; $—C(OR^5)(OR^6)—$, $—C(OR^5)(SR^6)—$ ou $—C(SR^5)(SR^6)—$, où $R^5$ et $R^6$ représentent chacun indépendamment un groupe alcoyle ou représentent ensemble un groupe alcoylène; $—CH(CH_2COOR^7)—$ où $R^7$ représente un groupe alcoyle; ou $—(CNR^8)—$ où $R^8$ représente un groupe $OR^4$ ou $HNR^4$; et l'autre de A et B représente un groupe $—CR^9R^{11}—CR^{10}R^{12}—$, où chacun de $R^9$, $R^{10}$, $R^{11}$ et $R^{12}$ indépendamment représente un atome d'hydrogène ou un groupe alcoyle, ou $R^{11}$ et $R^{12}$ ensemble représentent une liaison carbone-carbone, un groupe $—CH_2—$ ou un atome d'oxygène; mais ne comprenant pas les composés de formule I dans lesquels $R^1$, X et Y sont tous des atomes d'hydrogène, Ar est un groupe phényle non substitué et la liaison —A—B— est $—CO—CH(CH_3)—CH_2—$ ou $—(CH_2)_2—CO—$.

2. Un composé selon la revendication 1, dans lequel $R^1$ représente un groupe méthyle ou un atome d'hydrogène.

3. Un composé selon l'une quelconque des revendications 1 ou 2, dans lequel Ar représente un noyau de pyridine non substitué ou un groupe phényle qui est non substitué ou est substitué par un ou plusieurs substituants choisis parmi des atomes d'halogènes et des groupes alcoyle, alcoxy et haloalcoyle en $C_{1-4}$.

4. Un composé selon l'une quelconque des revendications 1 à 3, dans lequel X et Y représentent tous deux des atomes d'hydrogène.

5. Un composé selon l'une quelconque des revendications 1 à 4, dans lequel un de A et B représente un groupe $—CO—$, $—CR^3(OR^4)—$, $—C(OR^5)(OR^6)—$ ou $—C(SR^5)(SR^6)—$.

6. Un composé selon l'une quelconque des revendications précédentes, dans lequel $R^3$ représente un atome d'hydrogène.

7. Un composé selon l'une quelconque des revendications précédentes, dans lequel $R^4$ représente un atome d'hydrogène ou un groupe alcoyle en $C_{1-4}$, ($C_{1-4}$ alcoyl)-carbonyle ou méthylsulfonyle; et $R^5$ et $R^6$ représentent chacun indépendamment un groupe alcoyle en $C_{1-4}$ ou $R^5$ et $R^6$ représentent ensemble un groupe diméthylène ou triméthylène.

8. Un composé selon l'une quelconque des revendications précédentes, dans lequel un de A et B représente un groupe $—CO—$, $—CH(OH)—$, $—CH(OCH_3)—$ ou

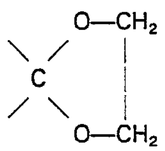

9. Un composé selon l'une quelconque des revendications 1 à 8, dans lequel un de A et B représente un groupe $—CH=CH—$ ou $—CH_2—CH_2—$.

10. Un composé selon l'une des revendications 5, 6 ou 7 et la revendication 8, dans lequel A représente le groupe de formule $—CO—$, $—CR^3(OR^4)—$, $—C(OR^5)(OR^6)—$ ou $—C(SR^5)(SR^6)—$ et B représente le groupe de formule $—CH=CH—$ ou $—CH_2—CH_2—$.

11. Un procédé pour la préparation d'un composé tel que revendiqué dans la revendication 1, qui comprend la réaction d'un composé de la formule générale:

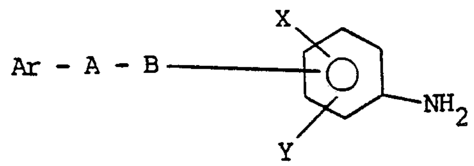

(II)

où Ar, A, B, X et Y ont les significations indiquées pour la formule générale I, avec soit

(a) un composé de la formule générale:

$$Hal—CO—N(CH_3)R^2 \qquad (III)$$

où $R^2$ a la signification indiquée pour la formule générale I et Hal représente un atome d'halogène; soit

(b) du phosgène, afin de produire l'isocyanate correspondant, et la réaction d'au moins une partie de l'isocyanate avec une amine de la formule générale:

$$NH(CH_3)R^2 \qquad (IV)$$

où $R^2$ a la signification indiquée pour la formule générale I; afin de produire un composé de la formule générale I où $R^1$ représente un atome d'hydrogène; et, si on le désire, la conversion du composé résultant de la formule générale I en n'importe quel autre composé désiré de la formule générale I.

12. Une composition herbicide et/ou fongicide qui comprend un composé selon l'une quelconque des revendications 1 à 10 en même temps qu'un véhicule.

13. Une composition selon la revendication 12, qui comprend au moins deux véhicules, dont au moins un est un agent tensio-actif.

14. Une méthode de lutte contre les champignons et/ou la croissance de plantes indésirables en un lieu, qui comprend l'application à ce lieu d'un composé de la formule générale

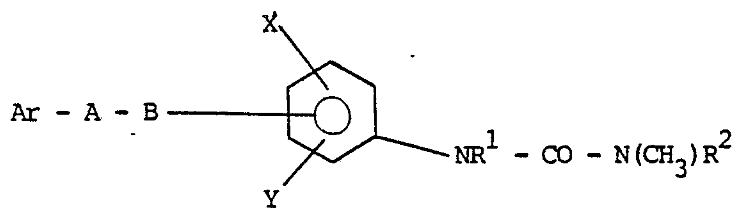

(I)

dans laquelle $R^1$ représente un atome d'hydrogène ou un groupe alcoyle ayant 1 à 4 atomes de carbone; $R^2$ représente un groupe méthyle; Ar représente un groupe phényle éventuellement substitué ou un noyau hétéroaromatique éventuellement substitué contenant 1 ou 2 atomes d'oxygène, de soufre et/ou d'azote, les substituants éventuels étant choisis parmi des atomes d'halogènes et des groupes alcoyle, alcoxy, haloalcoyle, haloalcoxy, alcoylthio, phényle, phénoxy, cyano, amino, alcoylamino, amido, hydroxy, carboxy et alcoxycarbonyle, les portions alcoyle dans ces substituants ayant jusqu'à 4 atomes de carbone; chacun de X et Y indépendamment représente un atome d'hydrogène ou d'halogène ou un groupe alcoyle, haloalcoyle ou alcoxy; et un de A et B représente un groupe —CO—; $—CR^3(OR^4)—$ ou $—CR^3(SR^4)—$ où $R^3$ représente un atome d'hydrogène ou un groupe alcoyle et $R^4$ représente un atome d'hydrogène, un groupe alcoyle, alcényle, alcynyle ou cycloalcoyle, un groupe phényle ou benzyle éventuellement substitué, les substituants éventuels étant choisis parmi des atomes d'halogènes et des groupes alcoyle, alcoxy, haloalcoyle, haloalcoxy, alcoylthio, phényle, phénoxy, cyano, amino, alcoylamino, amido, hydroxy, carboxy et alcoxycarbonyle, les portions alcoyle dans ces substituants ayant jusqu'à 4 atomes de carbone; ou un groupe acyle dérivé d'un acide carboxylique, carbamique, sulfonique ou phosphorique; $C(OR^5)(OR^6)—$, $—C(OR^5)(SR^6)—$ ou $—C(SR^5)(SR^6)—$, où $R^5$ et $R^6$ représentent chacun indépendamment un groupe alcoyle ou représentent ensemble un groupe alcoylène; $—CH(CH_2COOR^7)—$ où $R^7$ représente un groupe alcoyle; ou $—(CNR^8)—$ où $R^8$ représente un groupe $OR^4$ ou $HNR^4$; et l'autre de A et B représente un groupe $—CR^9R^{11}—CR^{10}R^{12}—$, où chacun de $R^9$, $R^{10}$, $R^{11}$ et $R^{12}$ indépendamment représente un atome d'hydrogène ou un groupe alcoyle, ou $R^{11}$ et $R^{12}$ ensemble représentent une liaison carbone-carbone, un groupe $—CH_2—$ ou un atome d'oxygène; mais, dans une méthode de lutte contre la croissance de plantes indésirables, n'est pas un composé de formule I dans lesquel $R^1$, X et Y sont tous des atomes d'hydrogène, Ar est un groupe phényle non substitué et la liaison —A—B— est $—CO—CH(CH_3)—CH_2—$ ou $—(CH_2)_2—CO—$.

**Revendications pour l'Etat contractant: AT**

1. Une composition herbicide et/ou fongicide qui comprend un ingrédient actif en même temps qu'un véhicule, caractérisée en ce que l'ingrédient actif est un composé de la formule générale

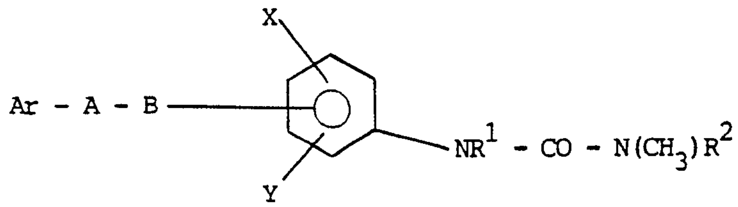

(I)

dans laquelle $R^1$ représente un atome d'hydrogène ou un groupe alcoyle ayant 1 à 4 atomes de carbone; $R^2$ représente un groupe méthyle; Ar représente un groupe phényle éventuellement substitué ou un noyau hétéroaromatique éventuellement substitué contenant 1 ou 2 atomes d'oxygène, de soufre et/ou d'azote, les substituants éventuels étant choisis parmi des atomes d'halogènes et des groupes alcoyle, alcoxy, haloalcoyle, haloalcoxy, alcoylthio, phényle, phénoxy, cyano, amino, alcoylamino, amido, hydroxy, carboxy et alcoxycarbonyle, les portions alcoyle dans ces substituants ayant jusqu'à 4 atomes de carbone; chacun de X et Y indépendamment représente un atome d'hydrogène ou d'halogène ou un groupe alcoyle, haloalcoyle ou alcoxy; et un de A et B représente un groupe —CO—; $—CR^3(OR^4)—$ ou $—CR^3(SR^4)—$, où $R^3$ représente un atome d'hydrogène ou un groupe alcoyle et $R^4$ représente un atome d'hydrogène un groupe alcoyle alcényle, alcynyle ou cycloalcoyle, un groupe phényle ou benzyle éventuellement substitué, les substituants éventuels étant choisis parmi des atomes d'halogènes et des groupes alcoyle, alcoxy, haloalcoyle, haloalcoxy, alcoylthio, phényle, phénoxy, cyano, amino, alcoylamino, amido, hydroxy, carboxy et alcoxycarbonyle, les portions alcoyle dans ces substituants ayant jusqu'à 4 atomes de carbone; ou un groupe acyle dérivé d'un acide carboxylique, carbamique, sulfonique ou phosphorique; $—C(OR^5)(OR^6)—$, $—C(OR^5)(SR^6)—$ ou $—C(SR^5)(SR^6)—$, où $R^5$ et $R^6$ représentent chacun indépendamment un groupe alcoyle ou représentent ensemble un groupe alcoylène; $—CH(CH_2COOR^7)—$ où $R^7$ représente un groupe alcoyle; ou $—(CNR^8)—$ où $R^8$ représente un groupe $OR^4$ ou $HNR^4$; et l'autre de A et B représente un groupe $—CR^9R^{11}—CR^{10}R^{12}—$, où chacun de $R^9$, $R^{10}$, $R^{11}$ et $R^{12}$ indépendamment représente un atome d'hydrogène ou un groupe alcoyle, ou $R^{11}$ et $R^{12}$ ensemble représentent une liaison carbone-carbone, un groupe $—CH_2—$ ou un atome d'oxygène; mais ne comprenant pas les composés de formule I dans lesquels $R^1$, X et Y sont tous deux atomes d'hydrogène, Ar est un groupe phényle non substitué et la liaison —A—B— est $—CO—CH(CH_3)—CH_2—$ ou $—(CH_2)_2—CO—$.

2. Une composition selon la revendication 1, dans laquelle $R^1$ représente un groupe méthyle ou un atome d'hydrogène.

3. Une composition selon l'une quelconque des revendications 1 ou 2, dans laquelle Ar représente un noyau de pyridine non substitué ou un groupe phényle qui est non substitué ou est substitué par un ou plusieurs substituants choisis parmi des atomes d'halogènes et des groupes alcoyle, alcoxy et haloalcoyle en $C_{1-4}$.

4. Une composition selon l'une quelconque des revendications 1 à 3, dans laquelle X et Y représentent tous deux des atomes d'hydrogène.

5. Une composition selon l'une quelconque des revendications 1 à 4, dans laquelle un de A et B représente un groupe —CO—, $—CR^3(OR^4)—$, $—C(OR^5)(OR^6)—$ ou $—C(SR^5)(SR^6)—$.

6. Une composition selon l'une quelconque des revendications précédentes, dans laquelle $R^3$ représente un atome d'hydrogène.

7. Une composition selon l'une quelconque des revendications précédentes, dans laquelle $R^4$ représente un atome d'hydrogène ou un groupe alcoyle en $C_{1-4}$, ($C_{1-4}$ alcoyl)-carbonyle ou méthylsulfonyle; et $R^5$ et $R^6$ représentent chacun indépendamment un groupe alcoyle en $C_{1-4}$ ou $R^5$ et $R^6$ représentent ensemble un groupe diméthylène ou triméthylène.

8. Une composition selon l'une quelconque des revendications précédentes, dans laquelle un de A et B représente un groupe —CO—, —CH(OH)—, $—CH(OCH_3)—$ ou

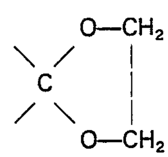

9. Une composition selon l'une quelconque des revendications 1 à 8, dans laquelle un de A et B représente un groupe —CH=CH— ou $—CH_2—CH_2—$.

10. Une composition selon l'une des revendications 5, 6 ou 7 et la revendication 8, dans laquelle A représente le groupe de formule —CO—, $—CR^3(OR^4)—$, $—C(OR^5)(OR^6)—$ ou $—C(SR^5)(SR^6)—$ et B représente le groupe de formule —CH=CH— ou $—CH_2—CH_2—$.

11. Une composition selon l'une quelconque des revendications précédentes, qui comprend au moins deux véhicules, dont au moins un est un agent tensio-actif.

12. Un procédé pour la préparation d'un composé de formule I, tel que défini dans la revendication 1, qui comprend la réaction d'un composé de la formule générale:

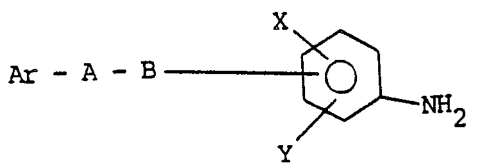

(II)

où Ar, A, B, X et Y ont les significations indiquées pour la formule générale I, avec soit

(a) un composé de la formule générale:

$$Hal—CO—N(CH_3)R^2 \qquad (III)$$

où $R^2$ a la signification indiquée pour la formule générale I et Hal représente un atome d'halogène; soit

(b) du phosgène, afin de produire l'isocyanate correspondant, et la réaction d'au moins une partie de l'isocyanate avec une amine de la formule générale:

$$NH(CH_3)R^2 \qquad (IV)$$

où $R^2$ a la signification indiquée pour la formule générale I; afin de produire un composé de la formule générale I où $R^1$ représente un atome d'hydrogène; et, si on le désire, la conversion du composé résultant de la formule générale I en un autre composé désiré quelconque de la formule générale I.

13. Une méthode de lutte contre les champignons et/ou la croissance de plantes indésirables en un lieu, qui comprend l'application à ce lieu d'une composition dans laquelle l'ingrédient actif est un composé de la formule générale

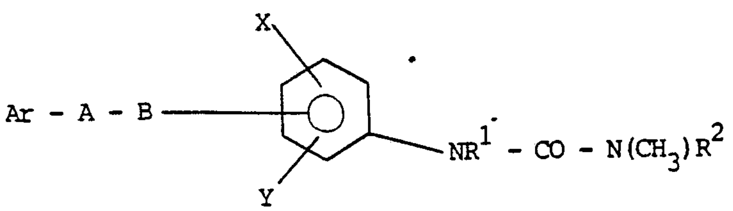

(I)

où $R^1$ représente un atome d'hydrogène ou un groupe alcoyle ayant 1 à 4 atomes de carbone; $R^2$ représente un groupe méthyle; Ar représente un groupe phényle éventuellement substitué ou un noyau hétéroaromatique éventuellement substitué contenant 1 ou 2 atomes d'oxygène, de soufre et/ou d'azote, les substituants éventuels étant choisis parmi des atomes d'halogènes et des groupes alcoyle, alcoxy, haloalcoyle, haloalcoxy, alcoylthio, phényle, phénoxy, cyano, amino, alcoylamino, amido, hydroxy, carboxy et alcoxycarbonyle, les portions alcoyle dans ces substituants ayant jusqu'à 4 atomes de carbone; chacun de X et Y indépendamment représente un atome d'hydrogène ou d'halogène ou un groupe alcoyle, haloalcoyle ou alcoxy; et un de A et B représente un groupe —CO—; $—CR^3(OR^4)—$ ou $—CR^3(SR^4)—$, où $R^3$ représente un atome d'hydrogène ou un groupe alcoyle et $R^4$ représente un atome d'hydrogène un groupe alcoyle, alcényle, alcynyle ou cycloalcoyle, un groupe phényle ou benzyle éventuellement substitué, les substituants éventuels étant choisis parmi des atomes d'halogènes et des groupes alcoyle, alcoxy, haloalcoyle, haloalcoxy, alcoylthio, phényle, phénoxy, cyano, amino, alcoylamino, amido, hydroxy, carboxy et alcoxycarbonyle, les portions alcoyle dans ces substituants ayant jusqu'à 4 atomes de carbone; ou un groupe acyle dérivé d'un acide carboxylique, carbamique, sulfonique ou phosphorique; $—C(OR^5)(OR^6)—$, $—C(OR^5)(SR^6)—$ ou $—C(SR^5)(SR^6)—$, où $R^5$ et $R^6$ représentent chacun indépendamment un groupe alcoyle ou représentent ensemble un groupe alcoylène; $—CH(CH_2COOR^7)—$ où $R^7$ représente un groupe alcoyle; ou $—(CNR^8)—$ où $R^8$ représente un groupe $OR^4$ ou $HNR^4$; et l'autre de A et B représente un groupe $—CR^9R^{11}—CR^{10}R^{12}—$, où chacun de $R^9$, $R^{10}$, $R^{11}$ et $R^{12}$ indépendamment représente une liaison carbone-carbone, un groupe $—CH_2—$ ou un atome d'oxygène; mais, pas les dans une méthode de lutte contre la croissance de plantes indésirables, n'est pas un composé de formule I dans lequel $R^1$, X et Y sont tous deux atomes d'hydrogène, Ar est un groupe phényle non substitué et la liaison —A—B— est $—CO—CH(CH_3)—CH_2—$ ou $—(CH_2)_2—CO—$.

**Revendications pour les Etats contractants: LU SE**

1. Un composé de la formule générale

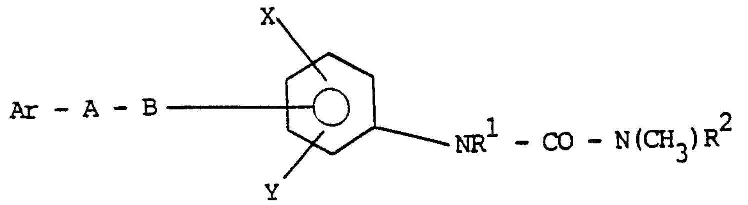

(I)

dans laquelle $R^1$ représente un atome d'hydrogène ou un groupe alcoyle ayant 1 à 4 atomes de carbone; $R^2$ représente un atome d'hydrogène ou un groupe methoxy ou méthyle; Ar représente un groupe phényle éventuellement substitué ou un noyau hétéroaromatique éventuellement substitué contenant 1 ou 2 atomes d'oxygène, de soufre et/ou d'azote, les substituants éventuels étant choisis parmi des atomes d'halogènes et des groupes alcoyle, alcoxy, haloalcoyle, haloalcoxy, alcoylthio, phényle, phénoxy, cyano, amino, alcoylamino, amido, hydroxy, carboxy et alcoxycarbonyle, les portions alcoyle dans ces substituants ayant jusqu'à 4 atomes de carbone; chacun de X et Y indépendamment représente un atome d'hydrogène ou d'halogène ou un groupe alcoyle, haloalcoyle ou alcoxy; et un de A et B représente un groupe —CO—; $—CR^3(OR^4)—$ ou $—CR^3(SR^4)—$, où $R^3$ représente un atome d'hydrogène ou un groupe alcoyle et $R^4$ représente un atome d'hydrogène, un groupe alcoyle, alcényle, alcynyle ou cycloalcoyle, un groupe phényle ou benzyle éventuellement substitué, les substituants éventuels étant choisis parmi des atomes d'halogènes et des groupes alcoyle, alcoxy, haloalcoyle, haloalcoxy, alcoylthio, phényle, phénoxy, cyano, amino, alcoylamino, amido, hydroxy, carboxy et alcoxycarbonyle, les portions alcoyle dans ces substituants ayant jusqu'à 4 atomes de carbone; ou un groupe acyle dérivé d'un acide carboxylique, carbamique, sulfonique ou phosphorique; $—C(OR^5)(OR^6)—$, $—C(CO^5)(SR^6)—$ ou $—C(CO^5)(SR^6)—$, où $R^5$ et $R^6$ représentent chacun indépendamment un groupe alcoyle ou représentent ensemble un groupe alcoylène; $—CH(CH_2COOR^7)—$ où $R^7$ représente un groupe alcoyle; ou $—(CNR^8)—$ où $R^8$ représente un groupe $OR^4$ ou $HNR^4$; et l'autre de A et B représente un groupe $—CR^9R^{11}—CR^{10}R^{12}—$, où chacun de $R^9$, $R^{10}$, $R^{11}$ et $R^{12}$ indépendamment représente un atome d'hydrogène ou un groupe alcoyle, ou $R^{11}$ et $R^{12}$ ensemble représentent une liaison carbone-carbone, un groupe $—CH_2—$ ou un atome d'oxygène.

2. Un composé selon la revendication 1, dans lequel $R^1$ représente un groupe méthyle ou un atome d'hydrogène.

3. Un composé selon l'une quelconque des revendications 1 ou 2, dans lequel Ar représente un noyau de pyridine non substitué ou un groupe phényle qui est non substitué ou est substitué par un ou plusieurs substituants choisis parmi des atomes d'halogènes et des groupes alcoyle, alcoxy et haloalcoyle en $C_{1-4}$.

4. Un composé selon l'une quelconque des revendications 1 à 3, dans lequel X et Y représentent tous deux des atomes d'hydrogène.

5. Un composé selon l'une quelconque des revendications 1 à 4, dans lequel un de A et B représente un groupe —CO—, $—CR^3(OR^4)—$, $—C(OR^5)(OR^6)—$ ou $—C(SR^5)(SR^6)—$.

6. Un composé selon l'une quelconque des revendications précédentes, dans lequel $R^3$ représente un atome d'hydrogène.

7. Un composé selon l'une quelconque des revendications précédentes, dans lequel $R^4$ représente un atome d'hydrogène ou un groupe alcoyle en $C_{1-4}$, ($C_{1-4}$ alcoyl)-carbonyle ou méthylsulfonyle et $R^5$ et $R^6$ représentent chacun indépendamment un groupe alcoyle ou $R^5$ et $R^6$ ensemble représentent un groupe diméthylène ou triméthylène.

8. Un composé selon l'une quelconque des revendications précédentes, dans lequel un de A et B représente un groupe —CO—, —CH(OH)—, $—CH(OCH_3)—$ ou

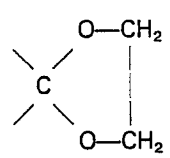

9. Un composé selon l'une quelconque des revendications 1 à 8, dans lequel un de A et B représente un groupe —CH=CH— ou $—CH_2—CH_2—$.

10. Un composé selon la revendications 5, 6 ou 7 et la revendication 8, dans lequel A représente le groupe de formule —CO—, $—CR^3(OR^4)—$, $—C(OR^5)(OR^6)—$ ou $—C(SR^5)(SR^6)—$ et B représente le groupe de formule —CH=CH— ou $—CH_2—CH_2$.

11. Un composé selon l'une quelconque des revendications précédentes, dans lequel $R^2$ représente un groupe méthyle.

12. Un procédé pour la préparation d'un composé de formule I, tel que défini dans la revendication 1, qui comprend la réaction d'un composé de la formule générale:

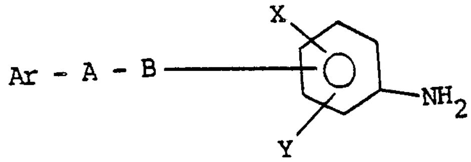

(II)

où Ar, A, B, X et Y ont les significations indiquées pour la formule générale I, avec soit

(a) un composé de la formule générale:

$$Hal—CO—N(CH_3)R^2 \quad (III)$$

où $R^2$ a la signification indiquée pour la formule générale I et Hal représente un atome d'halogène; soit

(b) du phosgène, afin de produire l'isocyanate correspondant, et la réaction d'au moins une partie de l'isocyanate avec une amine de la formule générale:

$$NH(CH_3)R^2 \quad (IV)$$

où $R^2$ a la signification indiquée pour la formule générale I; afin de produire un composé de la formule générale I où $R^1$ représente un atome d'hydrogène; et, si on le désire, la conversion du composé résultant de la formule générale I en un autre composé désiré quelconque un de la formule générale I.

13. Une composition herbicide et/ou fongicide qui comprend un composé selon l'une quelconque des revendications 1 à 11 en même temps qu'un véhicule.

14 Une composition selon la revendication 13, qui comprend au moins deux véhicules, dont au moins un est un agent tensio-actif.

15. Une méthode de lutte contre les champignons et/ou la croissance de plantes indésirables en un lieu, qui comprend l'application à ce lieu d'un composé selon l'une quelconque des revendications 1 à 10 ou d'une composition selon l'une des revendications 13 ou 14.